# EUROPEAN PATENT APPLICATION

(11) **EP 2 130 544 A2**
(43) Date of publication of application: **09.12.2009**
(21) Application number: 09011771.4
(22) Date of filing: 04.12.2006
(51) Int. Cl.: A61K 36/00, A61K 31/7034, C07H 1/08, A61P 35/00, A61P 5/30, A61P 3/06

(54) **Processes for obtaining lignan extracts and compositions containing the lignan extracts**

(30) Priority: 02.12.2005 US 742082 P; 31.05.2006 US 809652 P
(62) Divisional of application: 06844808.3
(71) Applicant: Archer-Daniels-Midland Company, Decatur, IL 62526 (US)
(72) Inventor: The designation of the inventor has not yet been filed
(74) Representative: Grünecker, Kinkeldey, Stockmair & Schwanhäusser Anwaltssozietät

(57) **Abstract**

Processes for obtaining lignan extracts from plant materials are disclosed. Compositions including lignan extracts as well as uses of the lignan extracts are also disclosed.

## Description

### TECHNICAL FIELD

The present invention is directed to processes of obtaining lignan extracts from plant materials. The invention is also directed to compositions containing the lignan extract obtained from the processes described herein as well as methods of using the compositions comprising the lignan extracts.

### BACKGROUND

Lignans are secondary plant metabolites, which are produced from shikimic acid via the phenylpropanoid pathway. They develop from flavonoid precursors and are responsible for conferring resistance to plants against pathogens and predators. Lignans are defined to be compounds possessing a 2,3-dibenzylbutane structure and include matairesinol, secoisolariciresinol, lariciresinol, isolariciresinol, nordihydroguaiaretic acid, pinoresinol, olivil and other compounds, and modifications thereof, including diglucosides.

Flaxseed (*Linum usitatissimum*) is potentially the richest source of the phytoestrogen lignans. The primary lignan found in flaxseed is 2,3-bis (3-methoxy-4-hydroxybenzyl) butane-1,4-diol (secoisolariciresinol) which is stored as the conjugate secoisolariciresinol diglucoside (SDG) in its native state in the plant. Flax seed contains levels of these phytoestrogens which are 75-800 times greater than any other plant food. The plant lignan, catecholic nordihydroguaiaretic acid, is a potent antioxidant previously used by the food industry.

Plant phenolic compounds occur as free monomers or in combination with other phytochemicals, thereby forming esters or glycosides. Phenolic acids are known to have antioxidant activity. The major phenolic constituents of flaxseed are reported to be coumaric acid (4-glucosyl-cinnamic acid), caffeic acid (3-hydroxy-4-glucosyl-cinnamic acid), ferulic acid (3-methoxy-4-glucosyl cinnamic acid) and hydroxy methyl glutaric acid. These compounds have antioxidant and hypercholesterermic properties.

Numerous reports in the literature have documented the phytochemical benefits of flaxseed lignans. Rickard et al. reported that feeding purified lignan at 5 percent flaxseed diet levels significantly reduces colon and mammary carcinogenesis in animals (Proceedings of the 57th Flax Institute of the United States, (Fargo, N.D.): 8-13 (1998)). Demark-Wahnefried et al. also reported that flaxseed supplementation may have a beneficial effect on prostate cancer biology (Demark-Wahnefried et al., Adult Urology 58(1): 47-52 (2001)).

Additionally, it has been reported that lignans prevent the development of Type I and Type II diabetes by 71 percent (Prasad, K. Proc. of the American Diabetes Association, (1999)), act as a hypotensive agent with ability to lower, blood pressure without affecting heart rate (US Pat. No. 6,498,145), provide benefits against Lupus Nephritis (U.S. Pat. No. 5,827,256), and reduce development of hypercholesterolemic atherosclerosis in animals (Atherosclerosis 132: 69-76 (1997)), along with numerous reports on the potential antioxidant (Mol. & Cell. Biochem., 202:91-100 (1999)) and anticancer properties (Anticancer Research 18:1405-1408 (1998)).

Flaxseed, in whole, ground or defatted form has been incorporated into animal feeds and food products such as breads, cookies, bagels and muffins. It has also been used for supplementing fiber levels in meat products (WO 00/19842). However, the amounts which can be used are regulated since high oil content of flax and the presence of mucilage contribute to excessive caloric intake and laxation (WO 96/30468).

Another problem associated with using flax in foods is the toxicity associated with cyanogenic glycosides present in flaxseed. Cyanogenic glycosides are nitrogenous secondary plant metabolites which if consumed in excess over a long period of time may result in goitrogenic problems, damage to human organs, cyanide toxicity or a possible thiocyanate effect of the thyroid. These glycosides are important natural toxins in both animal and human nutrition. They have been associated with flaxseed's unique property of protecting animals against the toxic effects of ingested selenium (Smith, et al., J. Org. Chem., 45:507-510 (1980)). The major cyanogenic glycosides present in flaxseed are linustatin, neolinustatin and linamarin, with linustatin accounting for 54-76 percent of the total cyanogenic glycoside content. Defatting of flax meal with hexane is known to produce an enrichment of all individual cyanogenic glycosides on equal weight basis in the meal (Maaza & Oomah, in Flaxseed in Human Nutrition, Cunnane & Thomson eds. AOCS Press, Champaign, III. 1995). Therefore, it is important to separate the cyanogenic glycosides from other compounds present in flax.

Methods for the preparation of lignans and other phenolic compounds have been reported in literature. In 1956, Bakke and Klosterman described a process for extracting lignan from defatted flax using methanol dioxane (Proceedings of the North Dakota Academy of Sciences 10:18-22 (1956)). However, lignans are known to occur as a "complex" in flax with cinnamic acid glucosides and other compounds. Hence, lignans have originally been referred to as a "polymer" in flax. Sodium and barium methoxides have been used for methanolysis to release lignans free of other compounds (Blake & Klosterman; Thompson et al., Nutr. Cancer 26:59-165 (1996)). Almost all of the lignans present in flaxseed occur as components of a soluble esterlinked complex and do not occur as free glycosides or aglycone. (Muir et al., Proc. of the 58th Flax Institute of the US, Fargo, N.D., 1999). A detailed review of the various methods of extracting lignan and cinnamic acids can be found in Muir, supra. U.S. Pat. No. 5,705,618 and PCT Applications WO 96/30468 and WO 00/78771 also describe methods of preparing lignan containing complexes. However, these processes suffer from drawbacks in that they cannot be scaled up for commercialization due to difficulty in achieving the desired separation and purity without involving complex solvent systems, such as ethyl acetate/water, and are difficult to separate using chromatographic techniques or employ expensive methods such as size exclusion chromatography. U.S. Pat. No. 6,767,565 describes a process for the purification of lignans using a membrane and resin chromatography process. However this process uses expensive resins and membranes that have poor stability in ethanol.

Thus, there exists a need for a more efficient process for isolating lignans as well as compositions including those lignans.

### SUMMARY OF THE INVENTION

In one embodiment, a composition comprises a lignan extract having from 20-45 percent secoisolariciresinol diglucoside. The composition also comprises 50 mg or less of cyanogenic glycosides per 100 grams of the composition.

In another embodiment, a composition comprises a first lignan extract having an amount of secoisolariciresinol diglucoside that is less than 50 percent and a second lignan extract having an amount of secoisolariciresinol diglucoside that is more than 50 percent.

In a further embodiment, a process for producing a lignan extract includes placing a lignan containing plant material in contact with an extraction solvent, thus forming an extraction solution. The process also includes separating a lignan enriched fraction from the extraction solution with a density based separation device, and washing the lignan enriched fraction with an aqueous solution. The process may further include drying the lignan enriched fraction.

Uses of the compositions or lignan extracts for reducing cholesterol levels, treating benign prostatic hyperplasia, treating heart disease, preventing hypercholesterolemic atherosclerosis, reducing heart disease, lowering serum cholesterol, reducing ischemic damage, increasing nitric oxide expression in endothelial cells, inhibiting cyclooxygenase activity in macrophages, alleviating symptoms associated with diabetes, preventing or delaying the onset of diabetes, modulating serum glucose levels, increasing bone density, reducing loss of bone density, alleviating symptoms associated with arthritis, reducing the risk of prostate cancer, reducing the risk of metastasis, delaying metastasis, alleviating symptoms associated with the onset of menstruation, alleviating symptoms associated with the onset of menopause, lowering blood pressure, or reducing hair loss or male pattern baldness are also disclosed. Uses of the compositions or extracts for manufacturing a medicament for treating these conditions is further described.

Further aspects of the invention are directed to compositions and formulations containing the lignan component obtained from the above described processes and methods of using the compositions and formulations.

### BRIEF DESCRIPTION OF THE DRAWINGS

Figures 1A through 1D are molecular weight distributions of lignan extracts produced using the process of the present invention.
Figure 2 depicts a flowchart illustrating acts in one embodiment of a process of extracting a lignan of the present invention.
Figures 3-4 are graphs illustrating the total cholesterol (TC) lowering ability of one embodiment of a lignan extract of the present invention.
Figures 5-6 are graphs illustrating the Low Density Lipoprotein Cholesterol (LDLC) lowering ability of one embodiment of a lignan extract of the present invention.
Figures 7-8 are graphs illustrating the High Density Lipoprotein Cholesterol (HDLC) raising ability of one embodiment of a lignan extract of the present invention.
Figures 9-10 are graphs illustrating the triglyceride (TG) lowering ability of one embodiment of a lignan extract of the present invention.
Figure 11 is a chart showing plasma levels of lignan metabolites in subjects treated with one embodiment of a lignan extract of the present invention.

### DETAILED DESCRIPTION OF THE INVENTION

Work on isolating or purifying lignan extracts has continued. US Patent 6,767,565 (the contents of the entirety of which is incorporated herein by this reference) assigned to Archer Daniels Midland Company of Decatur, Illinois, describes the use of membranes and resins to obtain lignan extracts from flaxseed. The present invention is directed towards an improved process for isolating or purifying lignan extracts. In various embodiments, the invention discloses processes for obtaining lignan extracts as well as compositions including the lignan extracts and uses thereof.

In one embodiment, a process may include contacting a lignan containing plant material with an extraction solvent to obtain an extraction solution comprising a lignan containing extract and the extraction solvent; separating plant material solids from said extraction solution; reducing microbial component content in the extraction solution; removing from 0 to 100 percent of the extraction solvent from the extraction solution, while simultaneously adding an aqueous diluent to the extraction solution; wherein a lignan is obtained therefrom.

In another embodiment, a process for obtaining lignan may comprise contacting a lignan containing plant material with an extraction solvent to obtain an extraction solution comprising a lignan containing extract and the extraction solvent; separating plant source solids from the extraction solution; reducing microbial component content in the extraction solution; contacting the extraction solution with an adsorptive resin; washing the adsorbed lignan containing extract with an aqueous diluent; eluting the lignan containing extract; thereby obtaining a lignan containing compound; and optionally, adjusting the pH of the released lignan before drying.

In a further embodiment, the process may also comprise a method for obtaining lignan from plant materials comprising: contacting a lignan containing plant material with an extraction solvent to obtain an extraction solution comprising a lignan containing extract and the extraction solvent; separating plant material solids from the extraction solution; reducing microbial component content in the extraction solution; removing from 0 to 100 percent of the extraction solvent from the extraction solution, while simultaneously adding an aqueous diluent to the extraction solution; wherein a lignan is obtained therefrom.

In one embodiment, a plant material such as flax, flaxseed or defatted flax may be pulverized by any suitable means such, as grinding, crushing and/or chopping. The pulverized plant material may be extracted with a suitable extraction solvent, such as an alcohol. The alcohol may be an aqueous alcohol or an aqueous aliphatic alcohol. A suitable alcohol may be selected from methanol, ethanol, isopropanol or any combinations thereof. In another embodiment an acetone water solution may be used. The concentration of the alcohol should be in the range of 50-80 percent by volume, as described by Westcott and Muir, "Medicinal Lignans from Flax Seed", Phytochemicals and Phyto-pharmaceuticals, AOCS Press, Champaign, III., 2000, and may be 60-70 percent by volume.

In certain embodiments where the raw material used is defatted flax meal, the pulverized material may be sifted and aspirated to removes fines created by the process as well as larger particles made up of the fines, which when put into the extractor will fall apart. Such fines may cause several problems in the subsequent extraction steps. The screening may be achieved using a combination of two different pore size screens with the middle portion being used for the production of the lignan extract. The act of sifting removes coarse material, which may contain trash, agglomerated fines and fatty lumps of soap stock present in defatted flax meal. In one embodiment, a first screen of 600 microns may be used in addition to a second screen of 2500 microns to screen the defatted flax meal. The portion of the flax meal that is retained between the two screens may be used for the production of the lignan extract. A fine screen may also be used to remove fine particles that plug the extractor screen and reduce percolation rates. The middle sifter cut may be aspirated and the "lights" fraction collected. The "lights" fraction of an aspirator is typically the one which provides more resistance to the movement of air and are therefore carried up with the airstream. Double aspiration is also sometimes done. The irregular shaped seed coat pieces collected in the lights fraction are higher in SDG relative to the denser particles which also offer less resistance to the air stream and are taken out as the "heavy" fraction. The irregular shaped seed coat pieces also prevent the extractor cake from packing tightly. The product of this process percolates well in liquid-solid extractor (such as those available from Crown Ironworks, Minneapolis MN) without plugging the screen used to drain the extracting solvent. Aspiration conditions be readily be determined by those skilled in the art and should ensure removal of the lower density/more air resistant fractions from the more dense/less air resistant particles.

In certain other embodiments, the plant material used to obtain the lignan extract may be selected from the group consisting of: pumpkin seeds; fiber-rich plants such as, for example, grains including, but not limited to, wheat, barley or oats; legumes such as beans, lentils or soybeans; and vegetables such as garlic, asparagus, broccoli or carrots.

The extraction may be carried out at a suitable temperature to minimize growth of potentially harmful microbial compounds and facilitate extraction of the lignan complex from the plant material. Suitable temperatures for minimizing the growth of microbes range from between room temperature and the boiling point of the solvent such as between 40° C and 70° C or between 50° C and 60° C.

The ratio of extraction solvent to the pulverized plant material should be suitable to permit agitation of the mixture. This ratio may be from 1:1 to 8:1 ml solvent per gram of pulverized plant material. Another ratio is from 2:1 to 6:1 ml solvent per gram of pulverized plant material.

The duration of the extraction process is from about 4-8 hours. In another embodiment, the extraction may be carried out from between about 5 and 7 hours. Where elevated temperatures are used, a 6 hour extraction time may be used. In another embodiment, the process may be carried out in a continuous mode in a current or counter current fashion as understood by those of ordinary skill in the art. The residence time may be varied to obtain the most suitable extraction from the flax meal. Such time may be determined by a time, temperature and ratio of extraction solvent to pulverized plant material. The time for extract may vary from 0.1 hours to 10 hours. In another embodiment, a residence time of 0.75 hours may be used in the counter current extractor.

The extraction process results in the formation of slurry comprising the extraction solvent, which contains the desired lignan component, and the solid plant material. After the extraction process is completed, the liquid fraction of the resulting slurry (hereinafter, extraction solution), which contains the desired lignan complex, may be separated from the solid plant material. The separation may be carried out in any suitable manner known for removing solids from a liquid. Examples of suitable techniques include, but are not limited to, filtration, precipitation, sedimentation, settling, centrifugation and any combinations thereof. Where filtration is used, the pore or mesh size of the membrane, filters, or screens is selected based on the size of the material being removed. The type of membrane filter selected is based on the compatibility of the membrane filter with the solvent used for the extraction process.

Where the plant material is flax, It has been observed that the flax swells during extraction and consequently absorbs a large amount of solvent at the end of extraction. The slurry, therefore, forms a wet meal cake that may be strained and/or pressed in order to remove the aqueous alcoholic extract from within the solid plant material component. Thus, in one embodiment, the wet meal cake may be pressed, such as with a screw press, to obtain the extraction solution. This substantially increases the yield of the lignan complex obtained from the flax wet meal cake.

In order to further increase the yield of the extraction solution, the screw press may also be used following separation of the plant material solids from the extraction solution where, for example, filtration, precipitation, sedimentation, settling and/or centrifugation is used as a separating technique.

The resulting extraction solution may be further clarified by passing the resulting extraction solution through a membrane filter to reduce the level of microbial components present in the solution and/or remove any residual solids not removed from the initial separation. One type of membrane filter for this clarification may be a nylon filter; however, any suitable type of filter may be used. In one embodiment, the percent reduction of microbial content is from about 90-100 percent, 90 percent, 95 percent, wherein about 98-100 percent is possible, and 99.9 percent is also possible.

The nylon membrane filter may have a pore size of from 0.1-10 µm or 0.2-10 µm. A membrane cartridge filter, having a similar pore size rating, may also be used. The resulting clarified extraction solution may be further processed according to, but not limited to, one of the embodiments described below.

The alcoholic extraction solvent may be removed (i.e., stripped) from the clarified extraction solution, such as by evaporation. It has been observed that the concentration of the extraction solution by evaporation results in the formation of a thick viscous layer which coalesces and settles quickly.

In order to avoid the effect of evaporating the extraction solvent and to allow for favorable processing of the desired extract, an aqueous diluent may be added to dilute the extraction solution during the evaporation/stripping of the extraction solvent. In one embodiment, the aqueous diluent may be water. Therefore, most of the extraction solvent present in the extraction solution can be removed if desired. In this way, the desired dilution of the extract may be maintained by the addition of water during the evaporation/stripping procedure.

In one embodiment, the resulting aqueous extraction solution (i.e., the aqueous supernatant layer) may be dried to obtain a powder. Drying may be carried out by any suitable method including, but not limited to, evaporation, vacuum drying, freeze drying, spray drying or combinations of any thereof.

In order to obtain a less hygroscopic product, in another embodiment, after reduction of the alcohol content in the clarified extraction solution by dilution and/or evaporation, the resulting extraction solution may be subjected to ultrafiltration in order to remove sugars (e.g. cyanogenic glycosides) present therein. Ultrafiltration may be carried out using at least two filter volumes of an aqueous diluent to remove cyanogenic sugars and other undesirable compounds. In one embodiment, the aqueous diluent may be water.

Suitable ultrafiltration membrane filters include organic polymers or copolymers, such as polysulfone, polyacrylonitrile, cellulose acetate, or inorganic materials such as zirconia, alumina or ceramic materials. Based on the molecular weight distribution of the desired lignan product, a membrane filter with a molecular weight cutoff (MWCO) of less than 5,000 may be used, or in another embodiment, a 1,000 MWCO membrane filter may be used.

Due to poor stability of polymeric membranes in high concentration alcohol solutions (Shukla, R. Ph.D. Thesis, University of Illinois at Urbana-Champaign, 2000), it may also possible to lower the alcohol concentration by dilution with water or by stripping the alcohol, such as by evaporation. Thus, the resulting reduced alcohol content extraction solution does not have a negative effect on the performance of the membrane filter.

The resulting ultrafiltered retentate is substantially free of cyanogenic sugars and contains the desired lignan extract in a relatively purified form. The ultrafiltered retentate may be dried directly or after further concentration by evaporation, by one or more of the following techniques: vacuum dryer, a microwave dryer, a radio frequency dryer, and/or a freeze-dryer. Vacuum drying for instance, may be accomplished at an absolute pressure of 5 - 20 milli bar, a residence time of 25 - 40 minutes and at a vapor temperature of 95 - 115 degrees F. Drying under these conditions may be accomplished with a Vacuum Belt Dryer.

Vacuum belt or band driers include a housing with built-in conveyer belts for conveying the product to be dried over heating plates. Automatic belt control ensures precision belt operation. Such dryers are available from Merk Process (Laufenberg, Germany). The belts run parallel to one another and a metering pump with an oscillating application nozzle is assigned to each individually controlled belt. The shift in the boiling point under vacuum provides for a low evaporation temperature and, therefore, a gentle product temperature. Pumpable products usually run through a highly viscous, frequently sticky phase during the drying process which may result in the formation of vapor bubbles in the product, as well as a dry cake being left on the belt toward the end of the drying process. The temperature to which the product is subjected during the drying process has a significant effect on the product quality. The belts run over a number of heating zones and over a cooling zone at the end. Steam, pressurized water or thermal oil can be used for heating, and each zone can be controlled separately, allowing the temperature of the heating surfaces to be adapted to the specific requirements of the product being dried. Utilizing such dryers reduces the loss in product quality and reduces the formation of undesirable color, as well as providing a free flowing shelf stable product.

In another embodiment, the extraction solution may be diluted with an aqueous diluent and instead of removing the extraction solvent, the diluted extraction solution may be subjected to ultrafiltration to remove sugars and other impurities.

In another embodiment, the invention discloses a process for obtaining a lignan extract comprising: contacting a lignan containing plant material with an extraction solvent to obtain an extraction solution comprising a lignan containing extract and the extraction solvent; separating plant source solids from the extraction solution; reducing microbial component content in the extraction solution; contacting the extraction solution with an adsorptive resin; washing the adsorbed lignan containing extract with an aqueous diluent; eluting the lignan containing extract; thus obtaining a lignan therefrom; and optionally, adjusting the pH of the released lignan before drying.

In another embodiment, the clarified extraction solution may be treated with an adsorptive resin in a batch or continuous column chromatography type process. The extract is applied to the adsorptive resin and washed with an aqueous diluent to remove cyanogenic sugars and other impurities. The aqueous diluent may be water.

The adsorbed lignan complex on the resin may be eluted with aqueous alcohol (20-100 percent by volume at 25-85° C) in a gradient or a single percentage process. The alcohol may be selected from, but is not limited to, methanol, ethanol, isopropanol or combinations of any thereof. The resulting material may be dried by, for example, evaporation, vacuum drying, freeze drying, spray drying or combinations of any thereof in order to produce a product which is approximately 20-45 percent lignan on a weight by weight basis. It has been found that adjusting the pH of the product of the chromatographic separation does not break down the lignan complex, but rather facilitates the drying of the product. The pH can be adjusted with any suitable acid or alkali reagent. In one embodiment, the act of pH adjusting may include adjusting the pH of the released lignan complex to between about 3.0 to about 9.0 before drying the product. In another embodiment, the pH may be adjusted from between about 7.5 to about 8.0.

Suitable resins that may be used are selected from, but are not limited to, polymethacrylate, ethylvinylbenze-divinylbenzene, styrene-divinylbenzene, polystyrene or phenol formaldehyde polymers, and may be either ionic or non-ionic.

In another embodiment, the extraction solution may be diluted with an aqueous diluent prior to treatment with the adsorptive resin. The aqueous diluent may be water.

In another embodiment, it may be desirable to adjust the pH of the extraction solution to a value of between about 3.0 to about 9.0. In one embodiment, the pH is between about 7.5 to about 8.0. It has been found that adjusting the pH accordingly with any suitable acid or alkali reagent does not break down the lignan complex, but rather facilitates the subsequent drying of the lignan product.

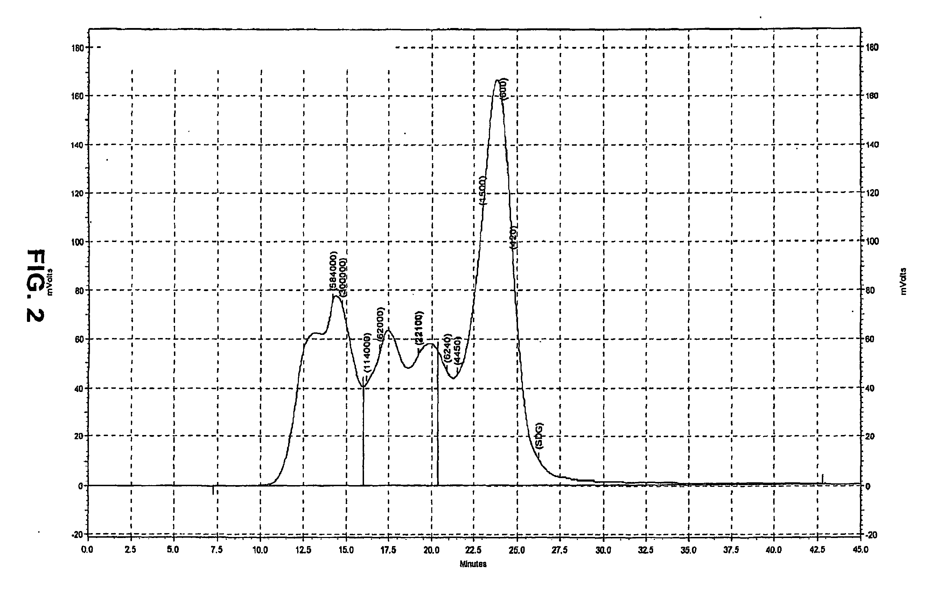

In another embodiment, a lignan extract is produced as described in the flowchart of Figure 2. Defatted flaxseed meal was obtained by removing oil from crushed flaxseed (*Linum uistatissimum*) using hexane extraction. The desolventized defatted flaxseed meal may optionally be mechanically (i.e., physically) classified to optimum particle size/shape. Such classification may be achieved by a combination of sifting and aspiration as described herein. The desolventized, and optionally classified, defatted flaxseed meal may be mixed with aqueous ethanol at 60-95 percent ethanol by volume at 45-75° C. The ethanol/flaxseed meal mixture may be filtered to remove any insolubles, resulting in an ethanol soluble extract.

The ethanol soluble extract may be evaporated under vacuum to remove substantially all of the ethanol. Depending on the temperature at which the evaporation takes place, the vacuum may be adjusted to enable the removal of the ethanol. The evaporated soluble extract containing the lignan complex may be separated from soluble and suspendable soluble impurities. In one embodiment, the separation of the lignan complex from the soluble and suspendable soluble impurities (i.e., non-lignan substances) may be done with a density based separation device or centrifugal device (e.g., a hydroclone or a continuous centrifuge) in order to coalesce and/or sediment out a viscous liquid enriched in the lignan complex, which may be decanted from a supernatant by separation with the centrifugal device. Such a process may also be referred to as sedimentation coalescence.

In another embodiment, the separation of the lignan complex from the soluble and suspendable soluble impurities may be done by precipitation, sedimentation or coalescence of the lignan complex. The use of a precipitation or settling tank is more cost effective as compared to the centrifugation or hydroclone type system as there are no mechanical parts involved. Further, the simplicity of the precipitation, sedimentation or coalescence provides a lower cost basis for manufacturing the product. The precipitation, sedimentation or coalescence may be done by pumping the evaporated soluble extract into a tank and allowing the lignan complex to sediment out, coalesce or fall to the bottom of the tank. In other embodiments, the precipitation, sedimentation or coalescence of the lignan complex may be aided by adjusting the pH. In yet another embodiment the separation of the lignan complex from the soluble and suspend able soluble impurities may be done with a filter. Any such filters known in the art may be used including, but not limited to, a polymeric or inorganic filter. The pore sizes of such filters may range from 0.01 microns to 100 microns.

The process intermediate containing the lignan complex may be washed by contacting the process intermediate with an aqueous citrate buffer. The act of washing the process intermediate removes cyanogenic glycosides from the enriched lignan complex (i.e., the process intermediate). The citrate buffer may comprise a 0.1 percent citrate buffer at a pH of about 5, or be up to about 10 percent citrate buffer. The pH may be varied in an acidic range such as a pH of 3.0 to 6.0 to maximize the removal of cyanogenic glycosides and minimize the losses of soluble lignans. In other embodiments, the acts of washing the lignan complex may also be performed with water, under neutral pH conditions, or alternatively a chelator including, but not limited to, ethylenediaminetetraacetate (i.e., EDTA).

The washed enriched lignan complex may be dried, optionally under vacuum, with or without preheating. The dried enriched lignan complex may be mechanically ground (i.e., using a Fitzmill, hammer mill or other similar device) to a specified size and may be packaged in polylined, poly drums for transport, placed in a food product, or placed in capsules or tablets. In one embodiment, the enriched lignan complex may be ground such that at least 90 percent of the enriched lignan is 425 µm (micro meters) or smaller. The evaporated ethanol, may be recycled or re-used without rectification by combining condensed vapors from the ethanol evaporation and adjusting the proof of the ethanol solution if desired.

In an additional embodiment, the lignan extract may be configured for oral administration to a subject such as in the form of a food additive, a beverage additive, a tablet, a capsule, a multi-ingredient nutritional supplement or any combination thereof. For instance, the lignan extract may be added to flax seed oil supplements, fish oil supplements, omega-3 fatty acid supplements or other dietary supplements for human consumption. The lignan extract may be used directly as a food additive or mixed with a consumable carrier to be used as the food additive, a food composition, a beverage additive or a beverage composition. Non-limiting examples of food products include, but are not limited to, nutritional bars, cereals, snacks, chips, crackers, cookies, baked goods, dairy products, food bars, nougats or any other food products. The lignan extract may also be mixed in or with a consumable food product such as a food composition for human consumption, livestock or an animal food.

It is also contemplated that the lignan extract may be prepared in capsule, tablet, caplet or liquid form for regular administration to help treat conditions associated with reducing cholesterol levels, treating benign prostatic hyperplasia, treating heart disease, preventing hypercholesterolemic atherosclerosis, reducing heart disease, lowering serum cholesterol, reducing ischemic damage, increasing nitric oxide expression in endothelial cells, inhibiting cyclooxygenase activity in macrophages, alleviating symptoms associated with diabetes, preventing or delaying the onset of diabetes, modulating serum glucose levels, increasing bone density, reducing loss of bone density, alleviating symptoms associated with arthritis reducing the risk of prostate cancer, reducing the risk of metastasis, delaying metastasis, alleviating symptoms associated with the onset of menstruation, alleviating symptoms associated with the onset of menopause, lowering blood pressure, or reducing hair loss or male pattern baldness. Beverages enriched for the lignan extract may be prepared for a like purpose, making the known benefits of lignans available to individuals. Beverages include, but are not limited to, apple juice, grape juice, cranberry juice, orange juice, energy drinks, sport drinks, meal replacement beverages, other fruit juices, any other soft drink, and dairy products such as milk, alcoholic beverages or combinations of any thereof.

In yet an additional embodiment, the lignan extract may be combined with any other compound or food additive such as sterols, isoflavones, sweeteners, protein isolates or concentrates, non-nutritive sweeteners, soluble or non-soluble fibers, statins, vitamin E, proanthocyanidins, saw palmetto extract, Pygeum extract, flax oil, fish oil, vitamin B, vitamin C, a tocopherol, a phytosterol, a polyphenol, a catechin, an anthocyanin, an astaxanthin, a glucosamine, and combinations of any thereof.

In yet a further embodiment, a lignan extract produced with one of the processes of the present invention may be used in a cosmetic product such as for a cosmetic, dermal or epidermal effect in an attempt to alter pigmentation. The cosmetic product may be configured for oral delivery as described herein or topical application such as with a lotion, sunscreen or other topical application.

The following non-limiting Examples are provided to further describe the invention. Those of ordinary skill in the art will appreciate that several variations these Examples are possible within the spirit of the invention and that various acts in the processes described may be employed in combination.

### EXAMPLES

### EXAMPLE 1.

A lignan extract was produced as described in the flowchart of Figure 2. Defatted flaxseed meal was obtained by removing oil from crushed flaxseed (*Linum uistatissimum*) using hexane extraction. The desolventized defatted flaxseed meal may optionally be mechanically (i.e., physically) classified to optimum particle size/shape as described herein. The desolventized, and optionally classified, defatted flaxseed meal was mixed with aqueous ethanol at 60-95 percent ethanol by volume at 45-75° C. The ethanol/flaxseed meal mixture may optionally be filtered to remove any insolubles, resulting in an ethanol soluble extract.

The ethanol soluble extract may also be evaporated under vacuum to remove substantially all of the ethanol. The precipitation or sedimentation may be done by pumping the evaporated soluble extract into a tank and allowing the lignan complex to coalesce and/or sediment out or fall to the bottom of the tank. Optionally a density based separation device such as a hydroclone or a centrifuge may also be used to achieve this separation. The process intermediate (Figure 2) containing the lignan complex may be washed by contacting the process intermediate with an aqueous citrate buffer. In an alternate embodiment a solution containing an organic acid such as acetic acid, fumaric acid, lactic acid, ascorbic acid may be used. The washed enriched lignan complex was dried, optionally under vacuum, with or without preheating, or optionally using a freeze dryer or a vacuum belt dryer. Table 1 provides an analysis of lignan extracts produced in this example.

Lignan extract obtained with the processes described herein comprises SDG or β-D-Glucopyranoside, (2R,3R)-2,3-bis[(4-hydroxy-3-methoxyphenyl) ]methyl]-1,4-butanediyl bis- (9Cl), having a formula of C₃₂H₄₆O₁₆ and a calculated molecular weight of about 686.7. The lignan extract may be in a powder form and may have a brown color, a "tea" like odor and taste, be slightly soluble in water at neutral pH and moderately soluble in 70 percent ethanol, and have a low hygroscopic character.

Five batches of lignan extracts were prepared with the process of this example. The lignan extract of the five batches had the following properties:

| **Table 1: Product analysis of 5 representative batches of lignan extracts.** | | | | | |
|---|---|---|---|---|---|
| **Batch Number** | **3A** | **4B** | **6A** | **6B** | **8A** |
| | | | | | |
| Total flax lignan (as SDG¹), percent as is | 36.1 | 36.6 | 37.8 | 37.4 | 36.3 |
| Protein (NX 6.25), as is | 3.2 | 4.7 | 3.2 | 2.3 | 3.6 |
| Fat, percent as is | 1.1 | 1.6 | 0.3 | 0.2 | 0.4 |
| Carbohydrate, percent by difference | 57.4 | 56.1 | 57.5 | 57.9 | 54.7 |
| Ash, percent | 0.4 | 0.3 | 0.4 | 0.3 | 0.2 |
| Moisture, percent | 1.8 | 0.7 | 0.8 | 1.9 | 4.8 |
| Arsenic, ppb | < 0.40* | < 0.40* | < 0.40* | < 0.40* | < 0.40* |
| Mercury, ppb | < 10.0* | < 10.0* | < 10.0* | < 10.0* | < 10.0* |
| Cadmium, ppm | < 0.05* | < 0.05* | < 0.05* | < 0.05* | < 0.05* |
| Lead, ppm | 0.32 | < 0.10* | < 0.10* | < 0.10* | < 0.10* |
| Cyanogenic glucosides³, g/kg | 0.50 | 0.39 | 0.39 | 0.09 | 0.14 |
| ¹ secoisolariciresinol diglucoside ³ Linamarin + Linustatin + Neolinustatin * Limit of detection. LOD | | | | | |

Lignan extracts produced with this example were also assayed for cyanogenic glycoside levels using methods described herein. It is typically recommended that the amount of cyanogenic glycosides consumed by a human be less than about 50 mg/adult/day. As shown in Table 1, the lignan extracts contain 50 mg or less of total cyanogenic glycosides per 100 g of lignan extract.

Various lignan enriched extracts (i.e., lignan extracts 1-6) were produced with the process of Example 1 and analyzed. The various analyzed products were determined to have the following properties:

| T**able 2: Analysis of lignan enriched extracts.** | | | | | | |
|---|---|---|---|---|---|---|
| **Compound** **All data is in g/kg** | **Whole Flaxseed** | **Lignan Extract 1** | **Lignan Extract 2** | **Lignan Extract 3** | **Lignan Extract 5** | **Lignan Extract 7** |
| Ash | 15.50 | 0.83 | 0.77 | 0.60 | 2.07 | 3.80 |
| Protein | 204.23 | 18.73 | 20.53 | 20.87 | 23.63 | 24.60 |
| Fat | 391.5 | 0.17 | 0.57 | 1.23 | 1.00 | 1.00 |
| Moisture | 80.4 | 53.5 | 47.9 | 45.0 | 40.5 | 35.5 |
| SDG | 9.3 | 363.9 | 373.32 | 373.12 | 368.84 | 370.31 |
| SDG isomer | 0.4 | 18.18 | 17.80 | 18.17 | 17.59 | 17.37 |
| Caffeic acid glucoside | 0.7 | 43.32 | 40.20 | 40.95 | 41.87 | 41.58 |
| Caffeic acid | 0.0 | 0.28 | 0.28 | 0.27 | 0.24 | 0.26 |
| p-coumaric acid glucoside | 2.6 | 162.50 | 143.73 | 146.19 | 149.18 | 148.30 |
| p-coumaric acid | 0.0 | 2.29 | 2.23 | 2.37 | 2.33 | 2.42 |
| Ferulic acid glucoside | 1.6 | 85.51 | 78.48 | 80.17 | 83.41 | 82.92 |
| Ferulic acid | 0.2 | 12.87 | 11.89 | 12.24 | 12.72 | 12.92 |
| 3,7-O-diglucosyl kaempferol | 0.0 | 2.31 | 2.22 | 1.38 | 1.13 | 1.40 |
| 3,8-O-diglucosyl herbacetin | 0.8 | 26.30 | 25.88 | 25.24 | 24.82 | 24.21 |
| Pinoresinol diglucoside | 0.4 | 17.12 | 16.76 | 16.47 | 15.77 | 16.37 |
| Astragalin (kaempferol-3-O-glucoside) | 0.1 | 2.49 | 2.46 | 2.38 | 2.28 | 2.47 |
| Ferulic acid derivative1 (has a molecular weight of about 552) | 0.5 | 18.59 | 18.76 | 18.82 | 18.50 | 18.50 |
| Ferulic acid derivative2 (has a molecular weight of about 552) | 0.3 | 13.49 | 13.65 | 13.66 | 13.47 | 13.31 |
| Total residue solvents | 0.0 | 0.094 | 0.074 | 0.102 | 0.081 | 0.131 |
| Total cyanogenic glucosides | 3.6 | 0.41 | 0.139 | 0.304 | 0.316 | 0.401 |
| Phytic acid | 6.3 | ND | ND | ND | ND | ND |
| Hydroxymethylglutaric acid (HMGA) | 2.6 | 123.60 | 121.62 | 131.52 | 125.80 | 128.94 |
| Quinic acid | 0.1 | 1.61 | 1.29 | 1.40 | 1.48 | 1.49 |
| Lactic acid | 0 | 3.80 | 3.98 | 4.29 | 4.09 | 4.24 |
| Acetic acid | 4.5 | 6.34 | 4.57 | 7.27 | 5.24 | 4.97 |
| Formic acid | 0.2 | 4.69 | 4.91 | 5.30 | 5.35 | 5.72 |
| Succinic acid | 0.5 | 22.10 | 25.45 | 22.22 | 22.95 | 24.44 |
| Malic acid | 0.4 | 38.23 | 26.64 | 37.38 | 33.41 | 38.42 |
| Glycerol | 5.34 | 0.82 | 0.97 | 1.19 | 0.87 | 0.50 |
| Citric acid | 5.20 | 0.66 | 0.59 | 0.75 | 0.00 | 0.00 |
| Myo-inositol | 1.28 | 0.30 | 0.37 | 0.46 | 0.11 | 0.00 |
| Sulfate | 0.4 | 0.25 | 0.29 | 0.30 | 0.30 | 0.38 |
| Chloride | 0.2 | 0.38 | 0.66 | 0.96 | 0.46 | 0.41 |
| Net carbohydrates (total) | 120.7 | -39.1 | 19.0 | -1.3 | -45.8 | -10.5 |
| TOTAL | 860.08 | 1045.50 | 1027.99 | 1032.54 | 1019.79 | 1027.28 |
| Total carbohydrates phenol-sulfuric | 274.11 | 348.8 | 396.1 | 377.4 | 333.0 | 368.2 |
| Known carbohydrates from assayed components | 8.64 | 387.89 | 377.06 | 378.68 | 378.80 | 378.67 |

Various lignan enriched extracts (i.e., lignan extract 7) produced with the process of Example 1, whole flaxseed, defatted flax meal, classified flax meal, and two process intermediates were also analyzed. The various analyzed products were determined to have the following properties as shown in Table 3:

| **Table 3: Analysis of flax meals and process intermediates.** | | | | | | |
|---|---|---|---|---|---|---|
| Compound All data is in g/kg | Flax meal | Classified Meal | Process Intermediate Product 4 | Lignan Extract 4 | Process Intermediate Product 6 | Lignan Extract 6 |
| Ash | 60.8 | 53.73 | 18.27 | 1.10 | 195.37 | 3.50 |
| Protein | 356.23 | 307.63 | 34.93 | 23.20 | 36.47 | 23.60 |
| Fat | 7.53 | 7.53 | 3.87 | 0.73 | 21.17 | 2.07 |
| Moisture | 81.6 | 84.5 | 24.6 | 47.3 | 65.2 | 38.9 |
| SDG | 18.81 | 22.71 | 328.38 | 366.15 | 299.02 | 369.44 |
| SDG isomer | 1.01 | 1.44 | 15.02 | 17.11 | 14.18 | 17.35 |
| Caffeic acid glucoside | 1.85 | 2.18 | 36.96 | 40.98 | 33.63 | 41.72 |
| Caffeic acid | 0.02 | 0.02 | 0.27 | 0.24 | 0.29 | 0.23 |
| p-coumaric acid glucoside | 6.53 | 7.89 | 131.62 | 146.09 | 119.87 | 148.76 |
| p-coumaric acid | 0.12 | 0.17 | 2.15 | 2.35 | 1.79 | 2.35 |
| Ferulic acid glucoside | 3.91 | 4.75 | 73.66 | 81.62 | 67.22 | 83.10 |
| Ferulic acid | 0.47 | 0.50 | 11.06 | 12.70 | 9.72 | 12.95 |
| 3,7-O-diglucosyl kaempferol | 0.06 | 0.07 | 1.12 | 1.36 | 1.27 | 1.05 |
| 3,8-O-diglucosyl herbacetin | 1.50 | 1.78 | 22.63 | 24.08 | 20.37 | 24.69 |
| Pinoresinol diglucoside | 1.02 | 1.15 | 14.08 | 15.68 | 13.20 | 15.73 |
| Astragalin (kaempferol-3-O-glucoside) | 0.14 | 0.19 | 1.99 | 2.36 | 2.03 | 2.31 |
| Ferulic acid derivative1 | 0.97 | 1.15 | 16.68 | 18.17 | 15.23 | 18.39 |
| Ferulic acid derivative2 | 0.62 | 0.80 | 11.95 | 13.13 | 10.74 | 13.36 |
| Total residue solvents | 0.037 | 0.112 | 4.733 | 0.092 | 1.079 | 0.084 |
| Total cyanogenic glucosides | 7.181 | 6.430 | 9.020 | 0.356 | 11.428 | 0.388 |
| Phytic acid | 6.9 | 5.7 | ND | ND | ND | ND |
| Hydroxymethylglutaric acid (HMGA) | 5.65 | 6.39 | 96.81 | 123.09 | 132.02 | 126.42 |
| Quinic acid | 0.40 | 0.43 | 2.96 | 1.40 | 4.39 | 1.29 |
| Lactic acid | | | 4.26 | 3.95 | 7.21 | 3.90 |
| Acetic acid | 9.70 | 9.35 | 8.27 | 5.90 | 7.15 | 6.24 |
| Formic acid | 0.61 | 0.64 | 4.56 | 5.00 | 6.13 | 5.20 |
| Succinic acid | 1.28 | 1.15 | 16.42 | 22.40 | 20.50 | 22.32 |
| Malic acid | 1.04 | 0.76 | 16.67 | 34.07 | 20.72 | 36.16 |
| Glycerol | 2.78 | 2.32 | 1.97 | 0.94 | 1.54 | 0.73 |
| Citric acid | 5.66 | 4.84 | 2.71 | 0.73 | 3.02 | 0.00 |
| Myo-inositol | 0.91 | 0.66 | 1.16 | 0.33 | 0.94 | 0.15 |
| Sulfate | 0.76 | 0.68 | 0.23 | 0.25 | 0.27 | 0.29 |
| Chloride | 0.27 | 0.38 | 0.63 | 0.54 | 1.01 | 0.52 |
| Net carbohydrates (total) | 329.1 | 352.2 | 92.3 | 4.5 | 105.4 | -37.9 |
| TOTAL | 915.45 | 890.22 | 1011.98 | 1017.94 | 1249.50 | 1023.23 |
| Total carbohydrates phenol-sulfuric | 347.8 | 374.8 | 428.6 | 378.1 | 412.1 | 340.4 |
| Known carbohydrates from assayed components | 18.72 | 22.64 | 336.27 | 373.60 | 306.7 | 378.33 |

All of the analyzed products were hydrolyzed prior to analysis and the lignan and phenolic compounds were analyzed with HPLC. The act of hydrolysis included placing the product in a solution of 56 percent methanol in water containing 0.1 M NaOH and heating at 65°C for one hour, as well as neutralization with dilute acetic acid. Other measurements were performed using conventional analysis methods.

### EXAMPLE 2.

Once obtained from the above described methods, the lignan product may be incorporated into a food, beverage, nutraceutical or pharmaceutical formulation. The formulations may be prepared for any route of administration to humans or animals such as, for example, oral delivery formulations. In one embodiment, formulations comprising the obtained lignan extract may comprise from 15-25 wt. percent of the lignan extract obtained from any of the above described methods; 60-84 wt. percent of a filler component; and 1-25 wt. percent of a dietary supplemental nutrient.

In other embodiments, the lignan extract placed into a food or other composition may comprise from about 7 to about 90 percent SDG depending on the desired use of the lignan extract.

### EXAMPLE 3.

The lignan extract is placed in a capsule or tablet for administration to a subject. The capsule or tablet may be placed in a container such as a bottle associated with indicia or instructions directing a user of a recommended dosage of the lignan extract. In various embodiments, the recommended dosage may be about 86-860 mg of lignan extract per day, which is about 30-300 mg of SDG per day, or in another embodiment, about 86-1720 mg of lignan extract per day, which is about 30-600 mg of SDG per day. When such amounts of lignan extracts are combined with excipients, fillers or other ingredients, a recommended dosage of a dietary supplement may be about 250 mg to about 1.5 grams. Indicia or instructions may also be associated with the dietary supplement or a container containing the dietary supplement to indicate to the user that the lignan extract contains a small amount of cyanogenic glycosides such as 50 mg or less of total cyanogenic glycosides per 100 g of lignan extract.

Although the various embodiments described herein are indicated to contain a certain concentration of SDG, it will be apparent to one of ordinary skill in the art that the concentration of SDG in the lignan extract may vary. For instance, the analyzed lignan or SDG content of the plant material from which the lignan extract originates may vary and be affected by a number of factors including, without limitation, variety of the plant material, location of the plant material, crop year of the plant material, extraction method used to extract the lignan extract, or hydrolysis and analytical technique used to measure the SDG content. For example, the SDG content in flax products has been reported to contain between 29-1055 mg/100 g seed or between 200-1300 mg/100 g flaxseed diet. Further, the SDG content has been reported to range from 0.96-3.15 µmoles/g in different varieties of flax. Thus, using the various embodiments described herein, a lignan extract or composition having any desired concentration of SDG from about 7-89 percent may be obtained and incorporated into a food, a nutraceutical, or a pharmaceutical composition using the methods disclosed herein.

### EXAMPLE 4.

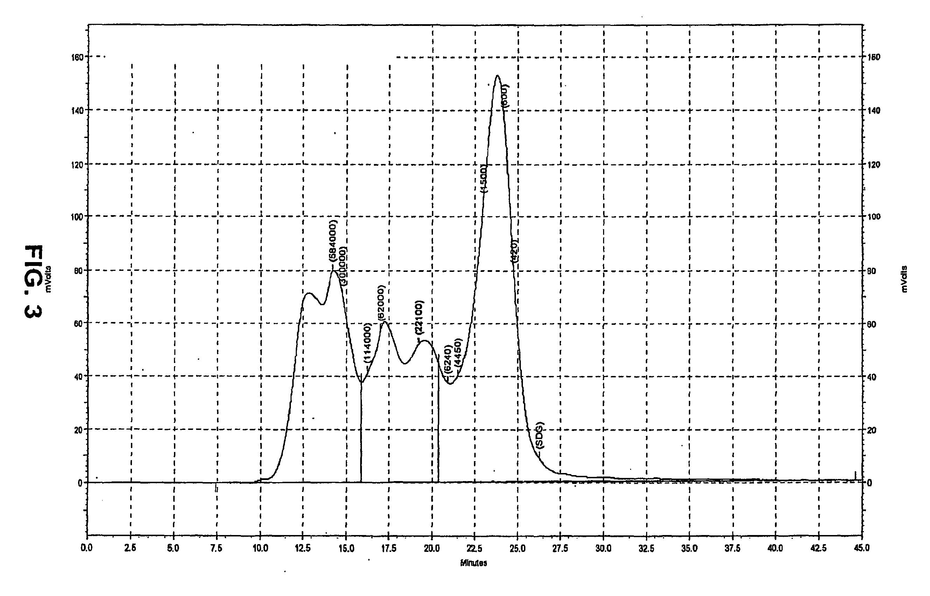

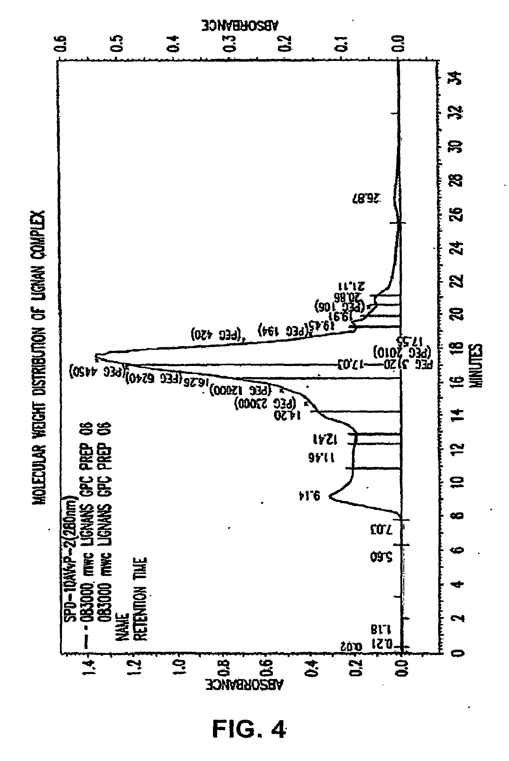

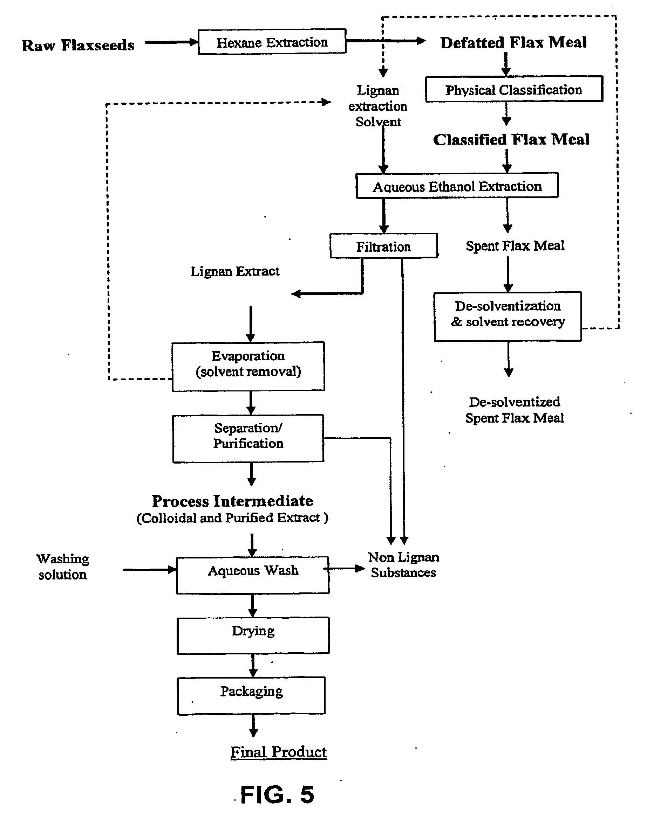

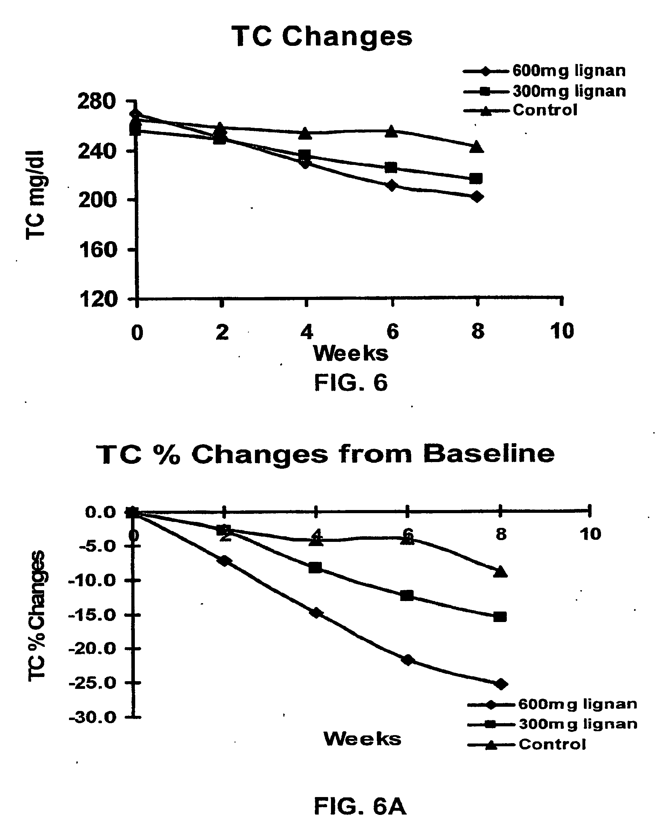

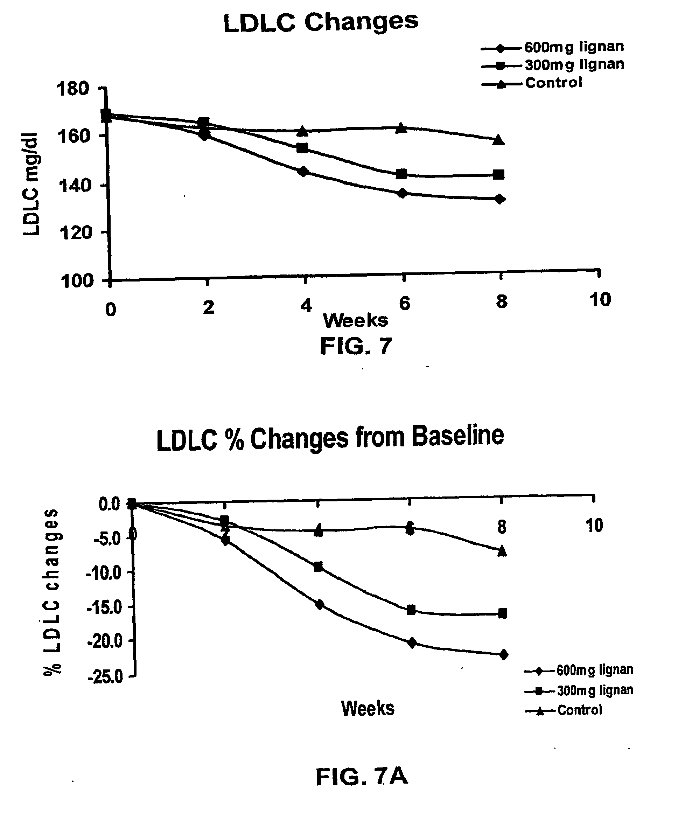

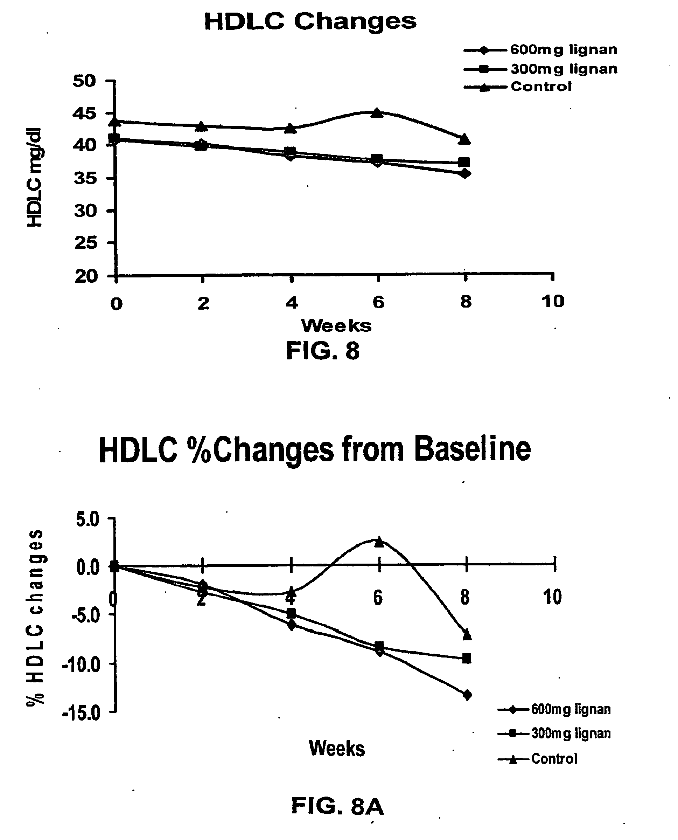

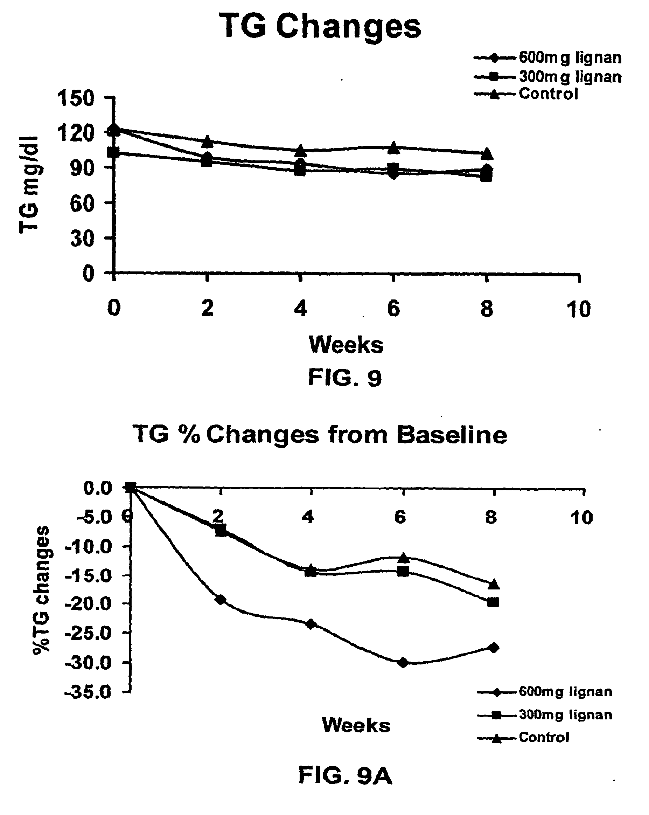

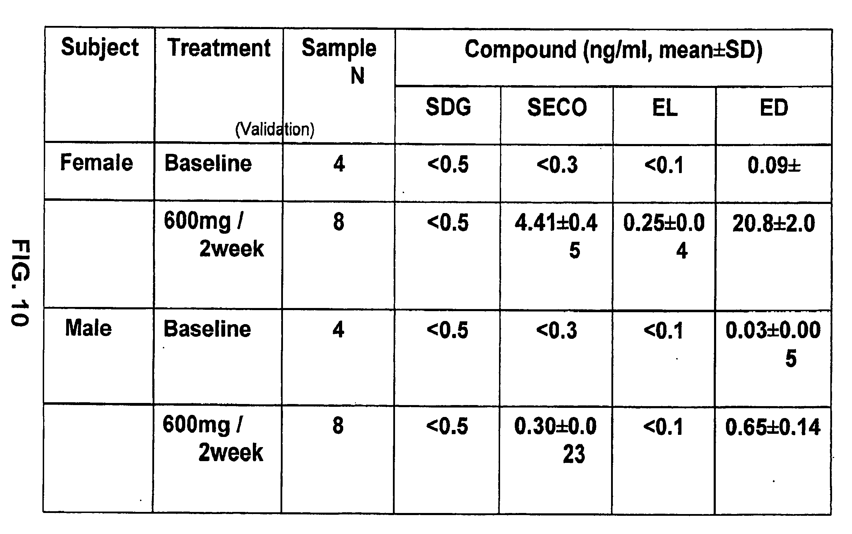

In another embodiment, the effect of a lignan extracted produced with the process of example 1 using centrifugation was studied in clinical trials. The clinical trials assayed plasma lipids in hypercholesterolemic subjects (20 women, 35 men, ages 28-79) using a randomized, placebo controlled double blind design for 8 weeks. The lignan extract was formulated into dietary supplement tablets in the following doses of SDG: 0 mg SDG (placebo), 300 mg SDG and 600 mg SDG administered to each subject per day. Total cholesterol (TC) and low density lipoprotein cholesterol (LDLC) were significantly reduced for the 300 mg and 600 mg SGD treatment doses when compared to the placebo as illustrated in Figures 3-6. High density lipoprotein cholesterol (HDLC) was also reduced as illustrated in Figures 7-8. Triglycerides (TG) levels were also reduced as illustrated in Figures 9-10. The plasma level of lignan metabolites in the subjects is illustrated in Figure 11.

The clinical trials also indicated that levels of creatine, blood urea nitrogen (BUN), alanine aminotransferase (ALT) and gamma glutamyl transferase (GGT) were observed to be similar among treatment groups and all these parameters are within normal range. The data is shown in Table 4 and indicates that the lignan extract had no noticeable effect on kidney or liver function.

| **Table 4: Safety Data of Lignan Extract in SDG-Cholesterol Lowering Clinical Trial.** | | | | | | | | | | |
|---|---|---|---|---|---|---|---|---|---|---|
| | | **Treatment: SFD=0** | | | **mg Treatment: SFD=300 mg** | | | **Treatment: SFD=600 mg** | | |
| **Variable** | **Week** | **N** | **Mean** | **Std Dev** | **N** | **Mean** | **Std Dev** | **N** | **Mean** | **Std Dev** |
| ALT (IU/L) | 0 | 18 | 18 | 4.99 | 17 | 18.65 | 6.14 | 20 | 18.25 | 6.34 |
| ALT (IU/L) | 4 | 18 | 21.11 | 5.27 | 16 | 19.63 | 4.76 | 20 | 20.15 | 4.25 |
| ALT (IU/L) | 8 | 15 | 19.8 | 3.53 | 16 | 21.25 | 4.28 | 18 | 23.22 | 4.99 |
| | | | | | | | | | | |
| BUN (mmol/L) | 0 | 18 | 4.53 | 1.31 | 17 | 4.54 | 1.13 | 20 | 3.89 | 1.14 |
| BUN (mmol/L) | 4 | 18 | 5.71 | 0.91 | 16 | 6.38 | 0.7 | 20 | 5.65 | 1.08 |
| BUN (mmol/L) | 8 | 15 | 5.42 | 1.27 | 16 | 5.07 | 1.35 | 18 | 4.53 | 1.65 |
| | | | | | | | | | | |
| Creatinine (mg/dl) | 0 | 18 | 0.94 | 0.16 | 17 | 0.89 | 0.12 | 20 | 0.93 | 0.14 |
| Creatinine (mg/dl) | 4 | 18 | 0.86 | 0.24 | 16 | 0.91 | 0.13 | 20 | 0.94 | 0.13 |
| Creatinine (mg/dl) | 8 | 15 | 0.96 | 0.11 | 16 | 0.94 | 0.14 | 18 | 0.92 | 0.15 |
| | | | | | | | | | | |
| GGT (IU/L) | 0 | 18 | 24.78 | 11.19 | 17 | 24.12 | 7.26 | 20 | 16.71 | 9.49 |
| GGT (IU/L) | 4 | 18 | 24.94 | 11.24 | 16 | 24.06 | 11.45 | 20 | 25.95 | 10.83 |
| GGT (IU/L) | 8 | 15 | 24.93 | 10.2 | 16 | 24.56 | 8.71 | 18 | 26.83 | 10.31 |

In this embodiment, the plasma levels of enterolignans (i.e., enterodiol and enterolactone) in subjects were also assayed. The plasma level of enterodiol was an average of 8.0 ng/ml in all subjects in the control group and 229.8 ng/ml in all subjects receiving 600 mg of SDG throughout the supplementation period (also a 28 fold difference), as depicted in the Table 5. The plasma levels of enterolignans suggest that administration of a lignan extract produced with the process of example 1 using centrifugation elevates the blood levels of enterolignans and enterodiols in human subjects.

| **Table 5: Plasma Levels of Enterolignans (Enterodiol and Enterolactone) in subjects supplemented with 300 and 600 mg/day of SDG (contained in Lignan extract) for eight weeks.** | | | |
|---|---|---|---|
| Treatment Dose (mg/day of SDG) | | | |
| Week | 0 (n=18) | 300 (n=17) | 600 (n = 20) |
| Plasma Enterodiol (ng/ml) | | | |
| 0 | 1.4 ± 2.3 | 10.7 ± 23.3 | 23.4 ± 58.9 |
| 2 | 6.4 ± 19.4 | 162.9 ± 194.6 | 266.9 ± 432.5 |
| 4 | 10.3 ± 25.6 | 124.9 ± 115.1 | 286.9 ± 438.2 |
| 6 | 7.2 ± 22.0 | 88.4 ± 71.9 | 132.7 ± 200.0 |
| 8 | 15.0 ± 38.9 | 103.4 ± 143.5 | 232.7 ± 432.0 |
| **X (Range)** | **8.1 (1.4-15.0)** | **119.8 (88.9-162.9)**** | **229.8 (132.7-266.9)**** |

| Enterolactone (ng/ml) | | | |
|---|---|---|---|
| 0 | 3.5 ± 3.7 | 13.7 ± 17.8 | 8.5 ± 18.5 |
| 2 | 7.7 ± 9.9 | 45.8 ± 54.9 | 56.9 ± 84.8 |
| 4 | 5.4 ± 7.0 | 35.7 ± 39.4 | 37.1 ± 58.7 |
| 6 | 3.8 ± 6.7 | 33.6 ± 54.3 | 34.5 ± 58.0 |
| 8 | 9.4 ± 11.6 | 55.5 ± 75.6 | 33.5 ± 44.2 |
| **X (Range)** | **6.0 (3.5-9.4)** | **42.2 (33.6-55.5)**** | **40.5 (33.5-56.9)**** |

* * Average value excludes baseline level to show the effect of SDG supplementation Vs, control group.

### EXAMPLE 5.

In a further embodiment, the effect of a lignan extract produced with a process of example 1 using centrifugation was studied in clinical trials. A study was performed to determine the effect of lignan extracts on benign prostatic hyperplasia (BPH) and prostrate specific antigen (PSA) in 60 men afflicted with the BPH condition. A randomized placebo controlled double blind design was used. The amount of lignan extract was formulated in dietary supplements to provide 0 mg lignan extract (placebo), 300 mg SDG in the lignan extract, or 600 mg SDG in the lignan extract in daily doses for a period of 16 weeks. Levels of creatine, blood urea nitrogen (BUN), alanine aminotransferase (ALT) and gamma glutamyl transferase (GGT) in blood were measured at 0, 2 and 4 months. Based on the results shown in Table 6, the administration of the lignan extract in this study did not affect kidney or liver function.

| **Table 6:Safety Data of Lignan Extract in SDG-BPH Clinical Trial** | | | | | | | | | | |
|---|---|---|---|---|---|---|---|---|---|---|
| | | **Treatment: 0 mg SDG** | | | **Treatment: 300 mg SDG** | | | **Treatment: 600 mg SDG** | | |
| **Variable** | **Month** | **N** | **Mean** | **Std Dev** | **N** | **Mean** | **Std Dev** | **N** | **Mean** | **Std Dev** |
| ALT (IU/L) | 0 | 22 | 18.82 | 9.54 | 25 | 20 | 6.48 | 23 | 23.26 | 11.36 |
| ALT (IU/L) | 1 | 9 | 20.56 | 7.55 | 13 | 27.69 | 10.13 | 11 | 22.91 | 8.54 |
| ALT (IU/L) | 2 | 22 | 15.45 | 5.34 | 25 | 19.04 | 10.75 | 23 | 22.52 | 11.04 |
| ALT (IU/L) | 3 | 22 | 19.55 | 7.19 | 24 | 27.13 | 16.16 | 23 | 27.65 | 11.3 |
| ALT (IU/L) | 4 | 21 | 17.38 | 7.9 | 24 | 21.54 | 7 | 22 | 23.27 | 9.56 |
| | | | | | | | | | | |
| BUN (mmol/L) | 0 | 22 | 6.39 | 1.96 | 25 | 5.68 | 1 | 23 | 5.99 | 1.47 |
| BUN (mmol/L) | 1 | 9 | 6.33 | 1.54 | 13 | 5.62 | 1.01 | 11 | 6.83 | 0.88 |
| BUN (mmol/L) | 2 | 22 | 6.1 | 1.68 | 25 | 5.75 | 1.5 | 23 | 5.91 | 1 |
| BUN (mmol/L) | 3 | 22 | 6.09 | 1.73 | 24 | 6.1 | 1.53 | 23 | 6.1 | 1.08 |
| BUN (mmol/L) | 4 | 21 | 6.36 | 1.88 | 24 | 5.85 | 1.31 | 22 | 5.77 | 1.31 |
| | | | | | | | | | | |
| Creatinine (µmol/L) | 0 | 22 | 79.91 | 14.41 | 25 | 82.6 | 16.02 | 23 | 80 | 18.55 |
| Creatinine (µmol/L) | 1 | 9 | 78.22 | 13.23 | 13 | 77.15 | 12.07 | 11 | 82.73 | 29.65 |
| Creatinine (µmol/L) | 2 | 22 | 76.64 | 13.83 | 25 | 81.6 | 16.39 | 23 | 75.09 | 19.63 |
| Creatinine (µmol/L) | 3 | 22 | 78.05 | 13.53 | 24 | 81.21 | 15.29 | 23 | 77.48 | 22.64 |
| Creatinine (µmol/L) | 4 | 21 | 74.14 | 15.06 | 24 | 79.33 | 15.81 | 22 | 79.68 | 27.43 |
| | | | | | | | | | | |
| GGT (IU/L) | 0 | 22 | 18.18 | 7.01 | 25 | 25 | 14.89 | 23 | 25.13 | 10.53 |
| GGT (IU/L) | 1 | 9 | 20 | 5.68 | 13 | 26.46 | 17.06 | 11 | 23.64 | 18.26 |
| GGT (IU/L) | 2 | 22 | 18.64 | 7.48 | 25 | 23.96 | 12.49 | 23 | 27.04 | 16.2 |
| GGT (IU/L) | 3 | 22 | 19.95 | 7.31 | 24 | 27.29 | 18.09 | 23 | 29.7 | 20.93 |
| GGT (IU/L) | 4 | 21 | 18.95 | 6.35 | 24 | 27.5 | 18.69 | 22 | 28.77 | 17.89 |

Significant relieving effects of the flax lignans on certain BPH symptoms were observed. Levels of creatinine, blood urea nitrogen (BUN), alanine aminotransferase (ALT) and gamma glutamyl transferase (GGT) in blood were measured and at 0, 8 and 16 weeks, no difference between and within treatment groups was observed. The results of this study also indicated that the lignan extract did not adversely affect kidney or liver function based on the normal levels of biomarkers as demonstrated in Table 7.

| **Table 7: Effect of Lignan Extract on Biomarkers of Kidney and Liver Function - BPH Study** | | | | | | | | | | | | |
|---|---|---|---|---|---|---|---|---|---|---|---|---|
| | | **Treatment: 0 mg SDG** | | | | **Treatment: 300 mg SDG** | | | | **Treatment: 600 mg SDG** | | |
| Variable | weeks | N | Mean | Std Dev | | N | Mean | Std Dev | | N | Mean | Std Dev |
| ALT (IU/L) | 0 | 22 | 18.82 | 9.54 | | 25 | 20 | 6.48 | | 23 | 23.26 | 11.36 |
| ALT (IU/L) | 4 | 9 | 20.56 | 7.55 | | 13 | 27.69 | 10.13 | | 11 | 22.91 | 8.54 |
| ALT (IU/L) | 8 | 22 | 15.45 | 5.34 | | 25 | 19.04 | 10.75 | | 23 | 22.52 | 11.04 |
| ALT (IU/L) | 12 | 22 | 19.55 | 7.19 | | 24 | 27.13 | 16.16 | | 23 | 27.65 | 11.3 |
| ALT (IU/L) | 16 | 21 | 17.38 | 7.9 | | 24 | 21.54 | 7 | | 22 | 23.27 | 9.56 |
| | | | | | | | | | | | | |
| BUN (mmol/L) | 0 | 22 | 6.39 | 1.96 | | 25 | 5.68 | 1 | | 23 | 5.99 | 1.47 |
| BUN (mmol/L) | 4 | 9 | 6.33 | 1.54 | | 13 | 5.62 | 1.01 | | 11 | 6.83 | 0.88 |
| BUN (mmol/L) | 8 | 22 | 6.1 | 1.68 | | 25 | 5.75 | 1.5 | | 23 | 5.91 | 1 |
| BUN (mmol/L) | 12 | 22 | 6.09 | 1.73 | | 24 | 6.1 | 1.53 | | 23 | 6.1 | 1.08 |
| BUN (mmol/L) | 16 | 21 | 6.36 | 1.88 | | 24 | 5.85 | 1.31 | | 22 | 5.77 | 1.31 |
| | | | | | | | | | | | | |
| Creatinine (µmol/L) | 0 | 22 | 79.91 | 14.41 | | 25 | 82.6 | 16.02 | | 23 | 80 | 18.55 |
| Creatinine (µmol/L) | 4 | 9 | 78.22 | 13.23 | | 13 | 77.15 | 12.07 | | 11 | 82.73 | 29.65 |
| Creatinine (µmol/L) | 8 | 22 | 76.64 | 13.83 | | 25 | 81.6 | 16.39 | | 23 | 75.09 | 19.63 |
| Creatinine (µmol/L) | 12 | 22 | 78.05 | 13.53 | | 24 | 81.21 | 15.29 | | 23 | 77.48 | 22.64 |
| Creatinine (µmol/L) | 16 | 21 | 74.14 | 15.06 | | 24 | 79.33 | 15.81 | | 22 | 79.68 | 27.43 |
| | | | | | | | | | | | | |
| GGT (IU/L) | 0 | 22 | 18.18 | 7.01 | | 25 | 25 | 14.89 | | 23 | 25.13 | 10.53 |
| GGT (IU/L) | 4 | 9 | 20 | 5.68 | | 13 | 26.46 | 17.06 | | 11 | 23.64 | 18.26 |
| GGT (IU/L) | 8 | 22 | 18.64 | 7.48 | | 25 | 23.96 | 12.49 | | 23 | 27.04 | 16.2 |
| GGT (IU/L) | 12 | 22 | 19.95 | 7.31 | | 24 | 27.29 | 18.09 | | 23 | 29.7 | 20.93 |
| GGT (IU/L) | 16 | 21 | 18.95 | 6.35 | | 24 | 27.5 | 18.69 | | 22 | 28.77 | 17.89 |

Summary results on the quality of life (QOL), international prostate symptom scores (IPSS), and urination blocking grade are presented in Tables 8-9. Compared to placebo, the 600 mg SDG treatment group obtained an overall significant improving effect on QOL scores (p=0.0115). For the 300 mg SDG treatment group, although the QOL scores were improved, statistical significance was not reached (p=0.1377). The percent changes of QOL scores from baseline had a similar statistical outcome as QOL score self (compared to placebo, p=0.0085 and =0.1248 for 600 and 300 mg SDG treatment, respectively). Significant treatment effects within subjects appeared in all groups. However, SDG treatment groups had stronger effects on QOL score lowering and smaller p values than did the placebo group.

The data of IPSS were collected based on seven questions related to a suffering experience of urination. Each question indicated a urinating symptom experienced by BPH patients. Each symptom was scored from 0 to 5. The higher score represents the symptom was more severe. Thus, the worst IPSS can be 35. In this study, the IPSS were significantly lowered in all groups. Between treatment groups, no overall significant effect was reached as compared to placebo. However, after 4 months of treatment, the IPSS of 300 and 600 mg SDG treatment groups were significantly lower than that of placebo group (p=0.002 and 0.018, respectively). The statistical outcomes of the percent changes from baseline were similar as that of IPSS changes. The percent IPSS lowering effect was somewhat significant in both of 300 and 600 mg SDG treatment groups (p=0.056 and 0.081, respectively) for the data of month 3 and 4; and the lowering effect was significant in the two treatment groups (p=0.0414 and 0.0412, respectively) for the data of month 4 alone. The noted significance was mainly because the IPSS-lowering effect of placebo group decreased after a period of time, meanwhile, the lowering effect of SDG treatment groups remained throughout the study.

Within group effect appeared in the placebo and the treatment groups. Compared to baseline, IPSS were significantly lowered at month 2, 3, or 4. The data of percent changes of IPSS from baseline are presented in Table 8. Very significant effects on IPSS lowering within group were obtained in both treatment groups, but not in placebo group (Table 8).

The urination blocking grade is used to specify difficulties of urination in BPH patients. The urination blocking grade includes scores of a group of urination symptoms with identical settings as IPSS. The blocking grade also contributes a part of the IPSS. Table 9 shows that significant treatment effects on blocking grade were obtained until month 4, as compared to placebo, p=0.019 and 0.012 for 300 and 600 mg SDG treatment groups, respectively. The reached significance was mainly due to the placebo effect on blocking grade lowering returning to baseline while and the effect of treatment groups remained. Within group effect on blocking grade lowering, the data of 300 and 600 mg SDG groups were all significantly lowered (p>0.005) as compared to baseline at month 2, 3, or 4. For placebo group, only the data at month 2 and 3 reached significant levels (p<0.05). The significant treatment effect on blocking grade lowering was observed only at month 4 (compared to placebo, p=0.024 and 0.013 for 300 and 600 mg SDG groups, respectively). The within group treatment effect strongly opened in both of 300 and 600 mg SDG treatment groups (p<0.005 for the data of every month). In placebo group, only the data at month 3 reached significant values (p<0.05).

| **Table 8: Percent changes of IPSS from baseline following lignan extract treatment** | | | | | |
|---|---|---|---|---|---|
| SDG (mg/day) | Month | N | Mean | Median | Std Dev |
| 0 | Baseline | 24 | 0 | 0 | 0 |
| | 2 | 23 | -23.27^{a} | -30.77 | 50.62 |
| | 3 | 23 | -24.68^{a} | -30 | 42.27 |
| | 4 | 21 | -9.53 | -21.74 | 61.97 |
| 300^{A} | Baseline | 27 | 0 | 0 | 0 |
| | 2 | 27 | -24.26^{a} | -33.33 | 33.63 |
| | 3 | 26 | -38.86^{b} | -40.83 | 22.14 |
| | 4 | 25 | -36.59^{b} | -35.71 | 27.63 |
| 600^{B} | Baseline | 23 | 0 | 0 | 0 |
| | 2 | 23 | -35.02^{b} | -34.38 | 31.59 |
| | 3 | 22 | -40.96^{b} | -49.52 | 28.45 |
| | 4 | 23 | -38.24^{b} | -50 | 36.94 |

| **Table 9: Percent changes of the blocking grade from baseline following flaxseed extract treatment** | | | | | |
|---|---|---|---|---|---|
| **SDG (mg/day)** | **Month** | **N** | **Mean** | **Median** | **Std Dev** |
| 0 | Baseline | 24 | 0 | 0 | 0 |
| | 2 | 23 | -22.55 | -50 | 60.77 |
| | 3 | 23 | -30.94^{a} | -40 | 39.14 |
| | 4 | 21 | -5.97 | -20 | 82.73 |
| 300^{A} | Baseline | 27 | 0 | 0 | 0 |
| | 2 | 27 | -27.36^{b} | -37.5 | 44.43 |
| | 3 | 26 | -38.63^{b} | -43.3 | 36.02 |
| | 4 | 24 | -37.18^{b} | -35.42 | 27.27 |
| 600^{B} | Baseline | 23 | 0 | 0 | 0 |
| | 2 | 23 | -35.20^{b} | -36.36 | 35.04 |
| | 3 | 22 | -39.32^{b} | -39.58 | 31.19 |
| | 4 | 23 | -42.76^{b} | -50 | 39.25 |

### EXAMPLE 6.

In another embodiment, the effect of a lignan extract produced by the process of example 1 using centrifugation was studied in clinical trials. A clinical trial was conducted to study the effect of phytosterols alone, or in combination with lignan extract on the effect of serum cholesterol in 25 men and 11 women for 8 weeks. A randomized placebo controlled, double blind design was used to assign the subjects to the following treatment groups: placebo, 1.3 g sterols, and 1.3 g sterols + 300 mg lignan extract. The levels of creatinine, blood urea nitrogen (BUN), alanine aminotransferase (ALT) and gamma glutamyl transferase (GGT) in blood were measured at 0, 2, 4, 6 and 8 weeks. In this embodiment, there was no difference in the concentration of these biomarkers between and within treatment groups indicating that the flax lignans do not adversely affect kidney or liver function as shown in Table 10A. The data shows that phytosterols in combinations with lignan extract lower total cholesterol by 13.5 percent and LDL-C by 18.9 percent as shown in Tables 10B-10D.

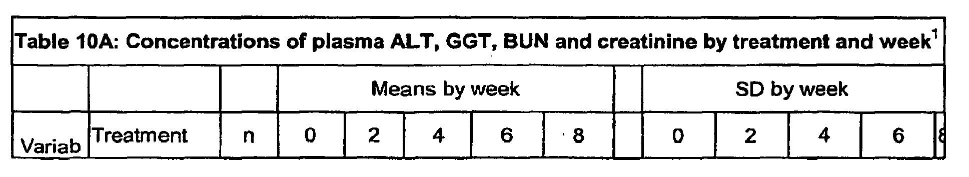

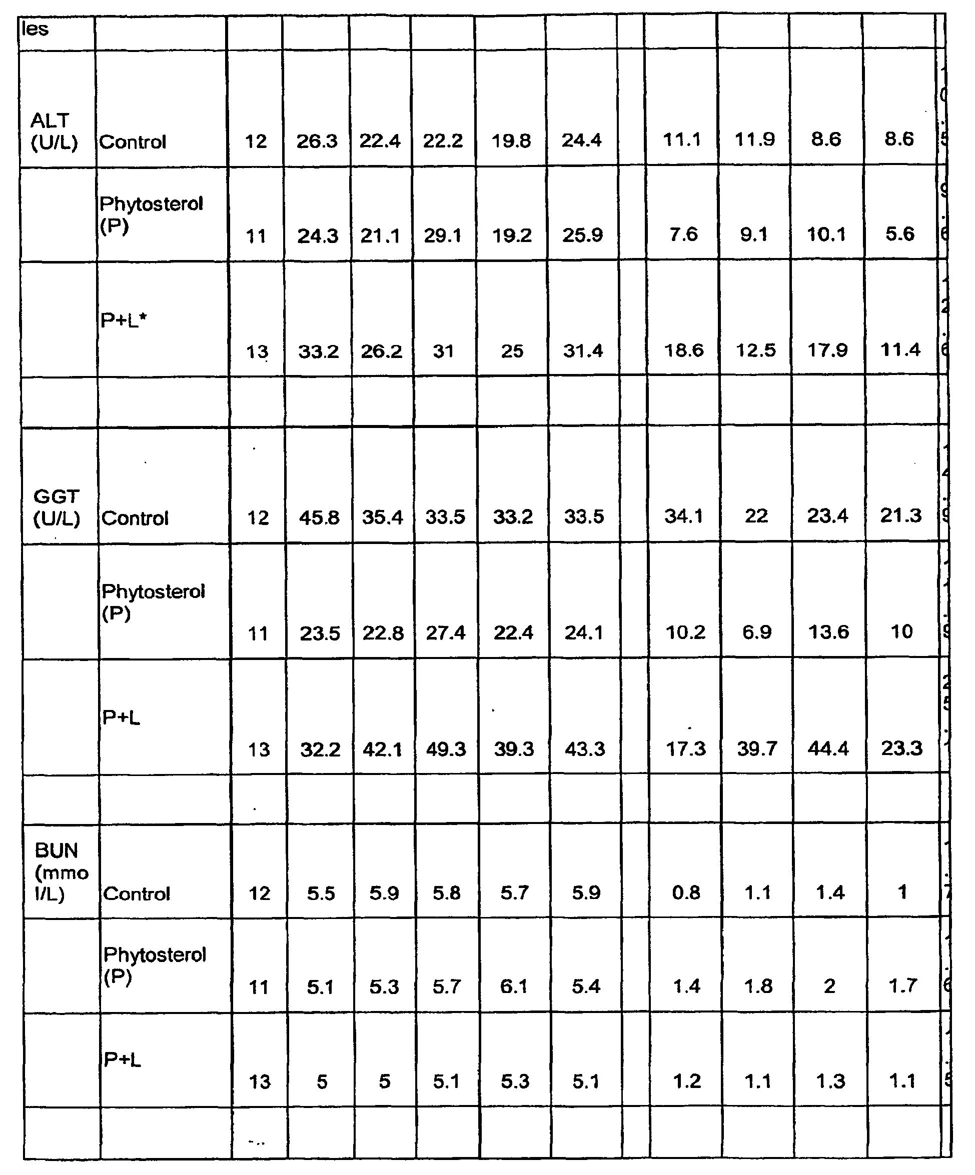

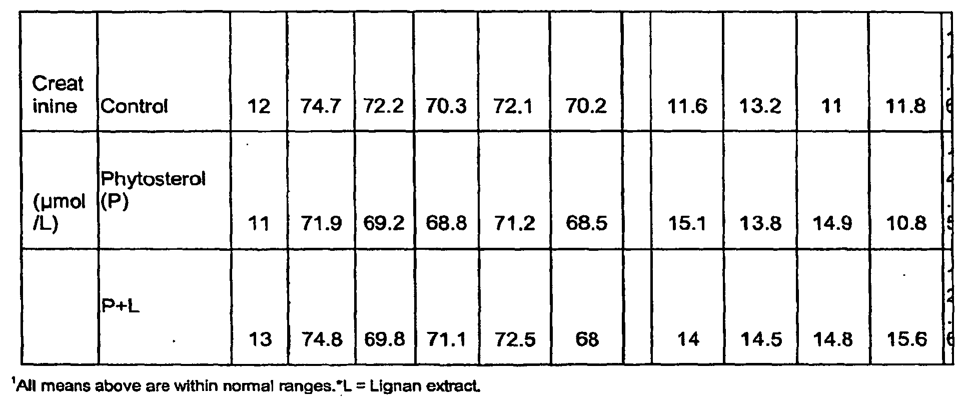

| **Table 10B: Treatment effects on plasma total cholesterol (TC).** | | | | | | | | | | | | |
|---|---|---|---|---|---|---|---|---|---|---|---|---|
| | | | Means by week | | | | | SD by week | | | | |
| | Treatment | n | 0 | 2 | 4 | 6 | 8 | 0 | 2 | 4 | 6 | 8 |
| TC concentrati ons | Control | 12 | 226. 2 | 218.4 | 212.3 | 218 | 209.4 | 23.6 | 26.9 | 29.2 | 24.2 | 24 .9 |
| (mg/dl) | Phytosterol (P) | 11 | 216. 3 | 204.8 | 204.1 | 202.7 | 207.1 | 17.1 | 31 | 33.7 | 22 | 24 .4 |
| | P+L* | 13 | 227. 5 | 204.9 | 200.9 | 207.4 | 196.1 | 24.4 | 30.4 | 33.6 | 28.4 | 35 .2 |
| | | | | | | | | | | | | |
| Changes from | Control | 12 | 0 | -7.8 | -13.9 | -8.1 | -16.7 ^{b} | 0 | 21.1 | 17.2 | 17.7 | 16 .5 |
| baseline (mg/dl) | Phytosterol (P) | 11 | 0 | -11.4 | -12.2 | -11.3 | -9.2 | 0 | 34.6 | 27.3 | 19.4 | 17 .8 |
| | P+L | 13 | 0 | -26^{a} | -26.6^{b} | -20.1^{a} | -31.4^{b} | 0 | 29.3 | 27.3 | 27.8 | 34 .3 |
| | | | | | | | | | | | | |
| %changes from | Control | 12 | 0 | -3.2 | -6.2^{a} | -3.4 | -7.3^{b} | 0 | 9.1 | 7.3 | 7.5 | 6. 7 |
| baseline | Phytosterol (P) | 11 | 0 | -4.8 | -5.8 | -5.2 | -4.3 | 0 | 15.4 | 12.5 | 8.8 8. | 8.2 |
| | P+L | 13 | 0 | -11^{b} | -11.6^{b} | -8.4^{a} | -13.5^{b} | 0 | 11.9 | 11.3 | 11.9 | 1 4 |

| | | | | | | | | | | | | |
|---|---|---|---|---|---|---|---|---|---|---|---|---|
| Compared to baseline, ^{a} = p<0.05; ^{b} = p<0:01. *L = Lignan extract | | | | | | | | | | | | |

| **Table 10C: Treatment effects on plasma total LDL cholesterol (LDL-C)** | | | | | | | | | | | | | |
|---|---|---|---|---|---|---|---|---|---|---|---|---|---|
| | | | Means by week | | | | | | SD by week | | | | |
| | Treatment | n | 0 | 2 | 4 | 6 | 8 | | 0 | 2 | 4 | 6 | 8 |
| LDLC | Control | 12 | 153.9 | 142.3 | 133.9 | 139.3 | 134.1 | | 20.7 | 18.5 | 23.6 | 21.6 | 23.2 |
| concentr ations | Phytosterol (P) | 11 | 154 | 132.6 | 134.7 | 134.3 | 137 | | 16.6 | 29.1 | 25.8 | 22.7 | 23.2 |
| (mg/dl) | P+L* | 13 | 153.5 | 133 | 128 | 130.6 | 123.8 | | 19.3 | 22.8 | 26.2 | 21.7 | 29.5 |
| | | | | | | | | | | | | | |
| Change s from | Control | 12 | 0 | -11.6 | -20^{b} | -14.6^{a} | -19.8^{b} | | 0 | 18.9 | 16.9 | 18.6 | 21.2 |
| baseline (mg/dl) | Phytosterol (P) | 11 | 0 | -21.5 | -19.3^{b} | -18.4^{a} | -17^{a} | | 0 | 30.2 | 19.2 | 18.7 | 16.8 |
| | P+L | 13 | 0 | -22.1^{b} | -25.5^{b} | -22.8^{b} | -29.7^{b} | | 0 | 18 | 18.3 | 22.2 | 29.6 |
| | | | | | | | | | | | | | |
| %chang es from | Control | 12 | 0 | -6.8 | -13^{b} | -9.1^{a} | -12.4^{b} | | 0 | 11.7 | 10.5 | 11.3 | 13 |
| baseline | Phytosterol (P) | 11 | 0 | -13.5 | -12.8^{b} | -12^{a} | -11.2^{a} | | 0 | 18.7 | 12.7 | 12.5 | 11.2 |
| | P+L | 13 | 0 | -14.2^{b} | -16.8^{b} | -14.4^{b} | -18.9^{b} | | 0 | 10.4 | 11.7 | 13.4 | 18.1 |

| | | | | | | | | | | | | | |
|---|---|---|---|---|---|---|---|---|---|---|---|---|---|
| Compared to baseline, ^{a} = p<0.05: ^{b} = p<0.01. *L = Lignan extract. | | | | | | | | | | | | | |

| **Table 10D: Treatment effects on plasma total HDL cholesterol (HDL-C)** | | | | | | | | | | | | | |
|---|---|---|---|---|---|---|---|---|---|---|---|---|---|
| | | | Means by week | | | | | | SD by week | | | | |
| | Treatment | n | 0 | 2 | 4 | 6 | 8 | | 0 | 2 | 4 | 6 | 8 |
| HDLC | Control | 12 | 49.3 | 43.3 | 43.9 | 47.5 | 45.5 | | 9.2 | 5.9 | 6.3 | 11.5 | 8.6 |
| concentr ations | Phytosterol (P) | 11 | 50.4 | 43.1 | 49 | 47.6 | 49.3 | | 10.7 | 11.7 | 12.2 | 11.7 | 10.5 |
| (mg/dl) | P+L* | 13 | 51.1 | 44.3 | 47.2 | 45.4 | 48.6 | | 11.3 | 8.6 | 11.2 | 9.9 | 9.8 |
| | | | | | | | | | | | | | |
| Changes from | Control | 12 | 0 | -6^{b} | -5.4^{a} | -1.8 | -3.7 | | 0 | 4.6 | 6.9 | 8.7 | 8.9 |
| baseline (mg/dl) | Phytosterol (P) | 11 | 0 | -7.3^{b} | -1.5 | -1.9 | -1.1 | | 0 | 6.1 | 6.6 | 5.1 | 3.9 |
| | P+L | 13 | 0 | -8^{b} | -3.8 | -5.7^{a} | -2.5 | | 0 | 6.2 | 5.8 | 8.5 | 9.8 |
| | | | | | | | | | | | | | |
| %chang esfrom | Control | 12 | 0 | -11.1 ^{b} | -9.7^{a} | -3.3 | -6.5 | | 0 | 7.4 | 11.9 | 17.1 | 15.3 |
| baseline | Phytosterol (P) | 11 | 0 | -15^{a} | -3 | -3.8 | -2 | | 0 | 13.4 | 11.8 | 8.9 | 6.9 |
| | P+L | 13 | 0 | -14.5^{b} | -7.3 | -9.8 ^{a} | -2.7 | | 0 | 9.9 | 10.8 | 14.5 | 20.5 |

| | | | | | | | | | | | | | |
|---|---|---|---|---|---|---|---|---|---|---|---|---|---|
| Compared to baseline, ^{a} = p<0.05; ^{b} = p<0.01. *L = Lignan extract | | | | | | | | | | | | | |

### EXAMPLE 7

In a further embodiment, in order to maintain a constant amount of SDG in the lignan extract, a first amount of a lignan extract such as the lignan extract obtained with the methods of the present invention, i.e., about 20-45 percent SDG, is mixed with a second amount of a lignan extract such as a substantially pure lignan extract, i.e., having at least 90 percent SDG. A lignan extract having 90 percent SDG is disclosed in US Patent 5,705,618 to Westcott et al., the contents of the entirety of which is incorporated by this reference.

Table 11 discloses compositions comprising various concentrations of SDG (37.75 percent - 88.8 percent SDG) that is produced by mixing a first lignan extract having about 35 percent SDG with a second lignan extract having about 90 percent SDG.

| **Table 11: Representative compositions of lignan extracts.** | | |
|---|---|---|
| Composition comprising Percent (%) SDG | First Extract 35% of SDG | Second Extract of 90% SDG |
| 37.75 | 9.5 g | 0.5 g |
| 40.5 | 9 g | 1 g |
| 46 | 8 g | 2 g |
| 51.5 | 7 g | 3 g |
| 57 | 6 g | 4 g |
| 62.5 | 5 g | 5 g |
| 68 | 4 g | 6 g |
| 73.5 | 3 g | 7 g |
| 79 | 2 g | 8 g |
| 84.5 | 1 g | 9 g |
| 87.25 | 0.5 g | 9.5 g |
| 88.8 | 0.2 g | 9.8 g |

### EXAMPLE 8

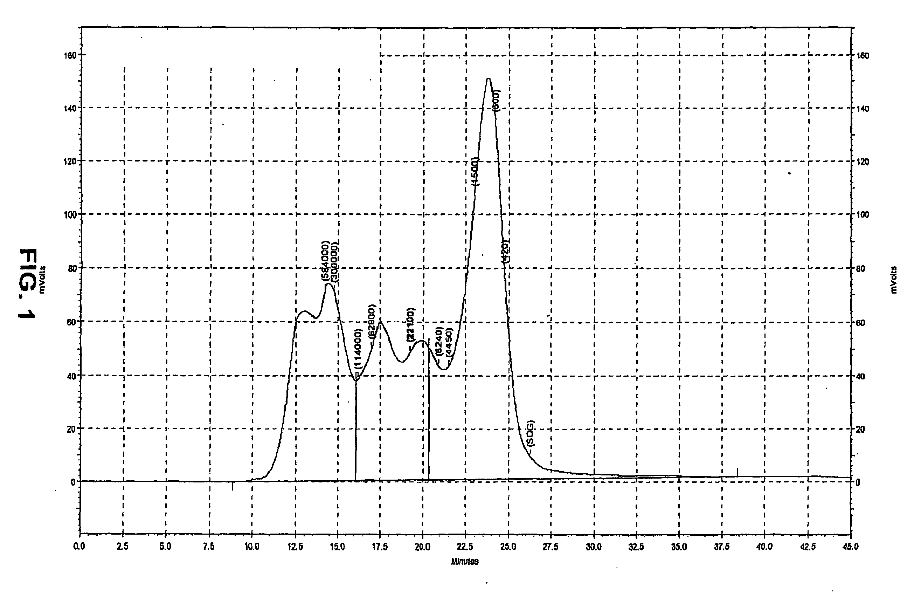

Molecular weights of lignan products produced by the process of example 1 using centrifugation were determined by Gel Permeation (Size Exclusion) Chromatography. Standard kits obtained from Phenomenex (Torrance, CA): Poly (ethylene glycol) part number AL0-2774 and Poly (ethylene oxide) part number AL0-2775 were used. Phenomenex PolySep-GFC-P 4000, 300 x 7.8 mm gel permeation chromatography column was used. A solvent mixture of 50 percent Acetonitrile / 50 percent water was used for elution at a flow rate 0.350 mL/min. Standards were determined by evaporative light scattering detection. Samples were determined by UV-Visible detection at 280 nanometers. Class-VP software Version 7.2.1 SP1 from Shimadzu Scientific Instruments, with the SEC (size exclusion chromatography) package, was used to calculate molecular weight parameters of the samples. These chromatograms are shown in Figures 1A-1D.

### EXAMPLE 9

Five lignan extracts produced by the embodiments of this invention were assayed for their constituents and the results are provided for in Table 12. Lots A-C are produced are using the embodiments of example 1 with coalescence/precipitation and lots D-E are produced using the embodiments of example 1 using centrifugation.

| Table 12 - Batch analyses of production and pilot lots of Lignan extract. | | | | | |
|---|---|---|---|---|---|
| Lignan extract | A | B | C | D | E |
| | | | | | |
| Total flax lignans (as SDG¹), % as is | 36.4 | 37.3 | 37.3 | 37.8 | 36.2 |
| Protein (N X 6.25), % as is | 1.88 | 2.05 | 2.09 | 3.2 | 2.6 |
| Fat, % as is | 0.02 | 0.06 | 0.12 | 0.3 | 0.4 |
| Carbohydrate², % | ND* | 1.9 | ND* | 4.77 | 6.72 |
| Ash, % | 0.08 | 0.08 | 0.06 | 0.4 | 0.2 |
| Moisture, % | 5.35 | 4.79 | 4.50 | 0.8 | 4.8 |
| SDG Isomer, % | 1.82 | 1.78 | 1.82 | NA⁺⁺ | NA⁺⁺ |
| Caffeic acid glucoside , % | 4.33 | 4.02 | 4.09 | 3.06 | 3.00 |
| Caffeic acid. % | 0.03 | 0.03 | 0.03 | 0.48 | 0.47 |
| Ferulic acid glucoside % | 8.55 | 7.84 | 8.01 | 6.56 | 6.45 |
| Ferulic acid % | 1.28 | 1.18 | 1.22 | 1.96 | 1.97 |
| Ferulic acid derivative #1.% | 1.85 | 1.87 | 1.88 | NA⁺⁺ | NA⁺⁺ |
| Ferulic acid derivative #2, % | 1.34 | 1.36 | 1.36 | NA⁺⁺ | NA⁺⁺ |
| p-Coumaric acid glucoside , % | 16.25 | 14.37 | 14.62 | 9.96 | 9.84 |
| p-Coumaric acid, % | 0.23 | 0.22 | 0.28 | 0.28 | 0.28 |
| 3,7-o-diglucosyl kaempferol, % | 0.04 | 0.22 | 0.14 | NA⁺⁺ | NA⁺⁺ |
| 3,8-o-diglucosyt herbacetin, % | 2.63 | 2.59 | 2.52 | NA⁺⁺ | NA⁺⁺ |
| Pinoresinol diglucoside, % | 1.71 | 1.68 | 1.65 | NA⁺⁺ | NA⁺⁺ |
| Astragalin, (kaempferol-3-O-glucoside),% | 0.25 | 0.25 | 0.24 | NA⁺⁺ | NA⁺⁺ |
| Total cyanogenic glucosides³, % | 0.041 | 0.014 | 0.030 | 0.025 | 0.014 |
| Phytic acid | ND* | ND* | ND* | NA⁺⁺ | NA⁺⁺ |
| Hydroxymethylglutaric acid (HMGA), % | 12.36 | 12.16 | 13.15 | 11.56 | 12.36 |
| Quinic acid, % | 0.16 | 0.13 | 0.14 | NA⁺⁺ | NA⁺⁺ |
| Lactic acid, % | 0.38 | 0.39 | 0.43 | 0.08 | 0.10 |
| Acetic acid, % | 0.63 | 0.46 | 0.73 | 0.34 | 0.34 |
| Formic acid, % | 0.47 | 0.49 | 0.53 | 0.12 | 0.12 |
| Succinic acid, % | 2.21 | 2.54 | 2.22 | 1.71 | 1.67 |
| Malic acid. % | 3.82 | 2.66 | 3.73 | 0.31 | 0.28 |
| Citric acid, % | 0.07 | 0.06 | 0.07 | 0.57 | 0.50 |
| Glycerol, % | 0.08 | 0.09 | 0.07 | NA⁺⁺ | NA⁺⁺ |
| Myo-inositol, % | 0.03 | 0.04 | 0.05 | NA⁺⁺ | NA⁺⁺ |
| Sulfate, % | 0.02 | 0.03 | 0.03 | NA⁺⁺ | NA⁺⁺ |
| Chloride, % | 0.04 | 0.07 | 0.10 | NA⁺⁺ | NA⁺⁺ |
| **Total** | **104.4** | **102.8** | **103.2** | **84.3** | **88.3** |
| | | | | | |
| ¹ Secoisolariciresinol diglucoside | | | | | |
| ² Net carbohydrate components excluding glycosides | | | | | |
| ³ Total = (Linamarin, Linustatin, Neolinustatin) | | | | | |
| ND = Not Detected (Below limit of detection for respective test). | | | | | |
| ⁺⁺ NA = Not analyzed | | | | | |

All publications mentioned herein are hereby incorporated in their entirety by reference.

While the forgoing invention has been described in some detail for purposes of clarity and understanding, it will be appreciated by one skilled in the art from a reading of this disclosure that various changes in form and detail can be made without departing from the scope of the invention and appended claims. For instance, while certain plant species, extracts, compositions, processes and uses thereof have been described, it will be recognized by those of ordinary skill in the art that various chemical substitutions or modifications of any of the exemplary embodiments may be made without departing from the spirit and scope of the invention. Further, any of the ingredients, compounds, compositions, or acts in processes of the invention may be made in combination.

What is disclosed is:
1. A composition comprising: a first lignan extract having an amount of secoisolariciresinol diglucoside that is less than 50 percent; and a second lignan extract having an amount of secoisolariciresinol diglucoside that is more than 50 percent.
2. The composition of embodiment 1, wherein the composition comprises 50-600 mg of the secoisolariciresinol diglucoside.
3. The composition of embodiment 1 or embodiment 2, wherein the composition is selected from the group consisting of a food, a beverage, a capsule, a tablet, and a caplet.
4. The composition of any one of embodiments 1-3, further comprising a compound selected from the group consisting of sterols, isoflavones, sweeteners, protein isolates, protein concentrates, non-nutritive sweeteners, soluble fibers, non- soluble fibers, statins, vitamin E, proanthocyanidins, saw palmetto extract, Pygeum extract, flax oil, fish oil, vitamin B, vitamin C, a tocopherol, a phytosterol, a polyphenol, a catechin, an anthocyan[iota]n, an astaxanthin, a glucosamine, and combinations of any thereof.
5. A composition comprising: a lignan extract having from 20-45% secoisolariciresinol diglucoside; wherein the composition comprises 50 mg or less cyanogenic glycosides per 100 g of the composition.
6. The composition of embodiment 5, wherein the composition comprises 300- 600 mg of the secoisolariciresinol diglucoside.
7. A container containing the composition of any one of embodiments 1-6, wherein the container is associated with indicia directing a user of a recommended dose of the composition.
8. The composition of embodiment 1, wherein the first lignan extract is 30 to 40 percent secoisolariciresinol diglucoside and the second lignan extract is at least 90 percent secoisolariciresinol diglucoside.
9. Use of the composition of any one of embodiments 1-6 or 8 in the manufacture of a medicament for reducing cholesterol levels, treating benign prostatic hyperplasia, treating heart disease, preventing hypercholesterolemic atherosclerosis, reducing heart disease, lowering serum cholesterol, reducing ischemic damage, increasing njtric oxide expression in endothelial cells, inhibiting cyclooxygenase activity in macrophages, alleviating symptoms associated with diabetes, preventing or delaying the onset of diabetes, modulating serum glucose levels, increasing bone density, reducing loss of bone density, alleviating symptoms associated with arthritis, reducing the risk of prostate cancer, reducing the risk of metastasis, delaying metastasis, alleviating symptoms associated with the onset of menstruation, alleviating symptoms associated with the onset of menopause, lowering blood pressure, or reducing hair loss or male pattern baldness.
10. A lignan extract comprising 46-89 percent secoisolariciresinol diglucoside.
11. Use of the lignan extract of embodiment 10 in the manufacture of a medicament for reducing cholesterol levels, treating benign prostatic hyperplasia, treating heart disease, preventing hypercholesterolemic atherosclerosis, reducing heart disease, lowering serum cholesterol, reducing ischemic damage, increasing nitric oxide expression in endothelial cells, inhibiting cyclooxygenase activity in macrophages, alleviating symptoms associated with diabetes, preventing or delaying the onset of diabetes, modulating serum glucose levels, increasing bone density, reducing loss of bone density, alleviating symptoms associated with arthritis, reducing the risk of prostate cancer, reducing the risk of metastasis, delaying metastasis, alleviating symptoms associated with the onset of menstruation, alleviating symptoms associated with the onset of menopause, lowering blood pressure reducing hair loss or male pattern baldness.
12. A method of reducing cholesterol levels, treating benign prostatic hyperplasia, treating heart disease, preventing hypercholesterolemic atherosclerosis, reducing heart disease, lowering serum cholesterol, reducing ischemic damage, increasing nitric oxide expression in endothelial cells, inhibiting cyclooxygenase activity in macrophages, alleviating symptoms associated with diabetes, preventing or delaying the onset of diabetes, modulating serum glucose levels, increasing bone density, reducing loss of bone density, alleviating symptoms associated with arthritis, reducing the risk of prostate cancer, reducing the risk of metastasis, delaying metastasis, alleviating symptoms associated with the onset of menstruation, alleviating symptoms associated with the onset of menopause, lowering blood pressure, reducing hair loss or male pattern baldness comprising administering the composition of any one of embodiments 1-6 or embodiment 8, or the lignan extract of embodiment 10 to a subject.
13. A method of reducing cholesterol levels, treating benign prostatic hyperplasia, treating heart disease, preventing hypercholesterolemic atherosclerosis, reducing heart disease, lowering serum cholesterol, reducing ischemic damage, increasing nitric oxide expression in endothelial cells, inhibiting cyclooxygenase activity in macrophages, alleviating symptoms associated with diabetes, preventing or delaying the onset of diabetes, modulating serum glucose levels, increasing bone density, reducing loss of bone density, alleviating symptoms associated with arthritis, reducing the risk of prostate cancer, reducing the risk of metastasis, delaying metastasis, alleviating symptoms associated with the onset of menstruation, alleviating symptoms associated with the onset of menopause, lowering blood pressure, reducing hair loss or male pattern baldness comprising administering a composition comprising a lignan complex having a molecular weight of at least 100,000 to a subject.
14. A method of treating benign prostatic hyperplasia comprising administering a lignan complex having a molecular weight of at least 100,000 to a subject.
15. A method of reducing serum cholesterol in humans comprising administering a lignan complex having a molecular weight of at least 100,000 to a subject.
16. A method of reducing blood pressure comprising administering a lignan complex having a molecular weight of at least 100,000 to a subject.
17. A method of reducing international prostate symptom scores comprising administering a lignan complex to a subject.
18. The method of anyone of embodiments 12-17, wherein the subject is a human.
19. The method of any one of embodiments 12-18, wherein the administration is done orally.
20. A process for producing a lignan extract, comprising: placing a lignan containing plant material in contact with an extraction solvent, thus forming an extraction solution; separating a lignan enriched fraction from the extraction solution with a density based separation device; washing the lignan enriched fraction with an aqueous solution; and drying the lignan enriched fraction.
21. The process according to embodiment 20, further comprising decanting a portion of the extraction solution away from the lignan enriched fraction.
22. The process according to any one of embodiments 20-21, wherein the aqueous solution comprises an aqueous citrate buffer.
23. The process according to any one of embodiments 20-22, further comprising removing a portion of the extraction solvent from the extraction solution.
24. The process according to embodiment 23, further comprising: wherein removing the portion of the extraction solvent from the extraction solution comprises evaporating the extraction solvent; and re-using the evaporated extraction solvent.
25. The process according to any one of embodiments 20-24, wherein the extraction solvent comprises an alcohol.
26. The process according to any one of embodiments 20-25, further comprising grinding the lignan enriched fraction.
27. The process according to any one of embodiments 20-26, further comprising packaging the lignan enriched fraction.
28. The process according to any one of embodiments 20-27, further comprising placing the lignan enriched fraction in a food product, a capsule, a tablet, a caplet, or a beverage.
29. The process according to any one of embodiments 20-28, wherein the lignan enriched fraction has a secoisolariciresinol diglucoside content of at least 35 percent by weight.
30. The process according to any one of embodiments 20-29, wherein the lignan enriched fraction comprises 50 mg or less of cyanogenic glycosides per 100 g of the lignan enriched fraction.
31. The process according to any one of embodiments 20-30, wherein the density based separation device is selected from a group consisting of a hydroclone, a settling tank, a centrifuge and a filter.
32. The process according to any one of embodiments 20-31, wherein the drying the lignan enriched fraction occurs in a dryer selected from the group consisting of a vacuum belt dryer, a microwave dryer, a radio frequency dryer, and a freeze-dryer.
33. The process according to any one of embodiments 20-32, further comprising an act selected from the group consisting of sifting the lignan containing plant material, aspirating the lignan containing plant material, or a combination thereof.
34. The process of embodiment 33, wherein two wire mesh screens are used to sift the lignan containing plant material.
35. The process of embodiment 34, wherein the two wire mesh screens comprise a 500 micron screen and a 2500 micron screen.
36. The process of any one of embodiments 20-35, further comprising mixing the lignan enriched fraction with a compound selected from the group consisting of sterols, isoflavones, sweeteners, protein isolates or concentrates, non-nutritive sweeteners, soluble or non-soluble fibers, statins, vitamin E, proanthocyanidins, saw palmetto extract, Pygeum extract, flax oil, fish oil, vitamin B, vitamin C, a tocopherol, a phytosterol, a polyphenol, a catechin, an anthocyanin, an astaxanthin, a glucosamine, and combinations of any thereof.
37. A product produced by the process of any one of embodiments 20-36.
38. Use of the product of embodiment 37 for reducing cholesterol levels, treating benign prostatic hyperplasia, treating heart disease, preventing hypercholesterolemic atherosclerosis, reducing heart disease, lowering serum cholesterol, reducing ischemic damage, increasing nitric oxide expression in endothelial cells, inhibiting cyclooxygenase activity in macrophages, alleviating symptoms associated with diabetes, preventing or delaying the onset of diabetes, modulating serum glucose levels, increasing bone density, reducing loss of bone density, alleviating symptoms associated with arthritis, reducing the risk of prostate cancer, reducing the risk of metastasis, delaying metastasis, alleviating symptoms associated with the onset of menstruation, alleviating symptoms associated with the onset of menopause, lowering blood pressure, or reducing hair loss or male pattern baldness.
39. Use of the product of embodiment 38 for the manufacture of a medicament for reducing cholesterol levels, treating benign prostatic hyperplasia, treating heart disease, preventing hypercholesterolemic atherosclerosis, reducing heart disease, lowering serum cholesterol, reducing ischemic damage, increasing nitric oxide expression in endothelial cells, inhibiting cyclooxygenase activity in macrophages, alleviating symptoms associated with diabetes, preventing or delaying the onset of diabetes, modulating serum glucose levels, increasing bone density, reducing loss of bone density, alleviating symptoms associated with arthritis, reducing the risk of prostate cancer, reducing the risk of metastasis, delaying metastasis, alleviating symptoms associated with the onset of menstruation, alleviating symptoms associated with the onset of menopause, lowering blood pressure or reducing hair loss or male pattern baldness.
40. A process for removing cyanogenic glycosides from a flax extract comprising: washing the flax extract with a compound selected from the group consisting of citric acid, ethylenediaminetetraacetate, acetic acid, fumaric acid, lactic acid and any combinations thereof.
41. The process of embodiment 40, wherein the compound has a pH of between pH 3.0-6.0.
42. A method for raising entero lactone, enterodiol or a combination thereof in the plasma of a subject comprising: administering an effective amount of the composition of any one of embodiments 1-6 or 8 or the product of embodiment 37 to the subject.
43. A method for raising enterolactone, enterodiol or a combination thereof in the plasma of a subject comprising: administering between 300mg/day and 600mg/day of the composition of any one of the embodiments 1-6 or 8 or the product of embodiment 37 to the subject.

## Claims

1. Use of a purified lignan complex having a molecular weight of at least 100,000 for the preparation of a medicament for reducing cholesterol levels, treating benign prostatic hyperplasia, treating heart disease, preventing hypercholesterolemic atherosclerosis, reducing heart disease, lowering serum cholesterol, reducing ischemic damage, increasing nitric oxide expression in endothelial cells, inhibiting cyclooxygenase activity in macrophages, alleviating symptoms associated with diabetes, preventing or delaying the onset of diabetes, modulating serum glucose levels, increasing bone density, reducing loss of bone density, alleviating symptoms associated with arthritis, reducing the risk of prostate cancer, reducing the risk of metastasis, delaying metastasis, alleviating symptoms associated with the onset of menstruation, alleviating symptoms associated with the onset of menopause, lowering blood pressure, reducing hair loss or male pattern baldness in a subject.

2. Use of a composition comprising a purified lignan complex having a molecular weight of at least 100,000 for the preparation of a medicament for treating benign prostatic hyperplasia in a subject.

3. Use of a purified lignan complex having a molecular weight of at least 100,000 for the preparation of a medicament for reducing serum cholesterol, blood pressure, or international prostate symptom scores in a subject.

4. The use of any one of claims 1-3, wherein the subject is a human.

5. The use of any one of claims 1-4, wherein the medicament is for oral administration.

6. The use of any one of claims 1-5, wherein the purified lignan complex is in a food product, a capsule, a tablet, a caplet, or a beverage.

7. The use of any one of claims 1-6, wherein the purified lignan complex has a secoisolariciresinol diglucoside (SDG) content of at least 35 percent by weight.

8. The use of any one of claims 1-7, wherein the purified lignan complex comprises 50 mg or less of cyanogenic glycosides per 100 g of the purified lignan complex.

9. The use of any one of claims 1-8, further comprising mixing purified lignan complex with a compound selected from the group consisting of sterols, isoflavones, sweeteners, protein isolates, or concentrates, non-nutritive sweeteners, soluble or non-soluble fibers, statins, vitamin E, proanthocyanidins, saw palmetto extract, Pygeum extract, flax oil, fish oil, vitamin B, vitamin C, a tocopherol, a phytosterol, a polyphenol, a catechin, an anthocyanin, an astaxanthin, a glucosamine, and combinations of any thereof.

10. The use of any one of claims 1-9, wherein upon administration of the purified lignan complex to the subject, levels of an enterolactone, an enterodiol, or a combination thereof are raised in the plasma of the subject.

11. The use of any one of claims 1-6 and 8-10, wherein the purified lignan complex comprises between 20-40% SDG and 33-39% phenol-sulfuric carbohydrates.

12. Use of a purified lignan complex having a molecular weight of at least 100,000 for the preparation of a medicament for reducing cholesterol levels, treating benign prostatic hyperplasia, treating heart disease, preventing hypercholesterolemic atherosclerosis, reducing heart disease, lowering serum cholesterol, reducing ischemic damage, increasing nitric oxide expression in endothelial cells, inhibiting cyclooxygenase activity in macrophages, alleviating symptoms associated with diabetes, preventing or delaying the onset of diabetes, modulating serum glucose levels, increasing bone density, reducing loss of bone density, alleviating symptoms associated with arthritis, reducing the risk of prostate cancer, reducing the risk of metastasis, delaying metastasis, alleviating symptoms associated with the onset of menstruation , alleviating symptoms associated with the onset of menopause, lowering blood pressure, reducing hair loss or male pattern baldness in a subject,
wherein the purified lignan complex comprises SDG, hydroxy methyl glutaric acid (HMGA), p-coumaric acid glycoside and caffeic acid glycoside.

13. The use of claim 12, wherein the purified lignan complex has a molecular weight of at least 114,000.

14. The use of any one of claims 12-13, wherein the subject is a human.

15. The use of any one of claims 12-14, wherein the purified lignan complex is in a food product, a capsule, a tablet, a caplet, or a beverage.
